# EUROPEAN PATENT SPECIFICATION

(11) **EP 4 453 236 B1**
(45) Date of publication and mention of the grant of the patent: **10.09.2025**
(21) Application number: 22854698.2
(22) Date of filing: 21.12.2022
(51) Int. Cl.: C12Q 1/66, G01N 33/58, G01N 33/68, G01N 33/542

(54) **METHODS AND SYSTEMS FOR ANALYZING TARGET ENGAGEMENT DATA FROM BIOLOGICAL ASSAYS**
VERFAHREN UND SYSTEME ZUR ANALYSE VON ZIELEINGRIFFSDATEN AUS BIOLOGISCHEN TESTS
PROCÉDÉS ET SYSTÈMES D'ANALYSE DES DONNÉES SUR L'ENGAGEMENT DES CIBLES DANS LES DOSAGES BIOLOGIQUES

(30) Priority: 22.12.2021 US 202163292919 P
(43) Date of publication of application: 30.10.2024
(73) Proprietor: The United States of America, as represented by the Secretary, Department of Health and Human Services, Maryland 20892-7788 (US)
(72) Inventor: RONZETTI, Michael H., Washington, D.C. 20010 (US); HENDERSON, Mark J., Damascus, Maryland 20872 (US); SANCHEZ, Tino W., Los Angeles, California 90065 (US); MICHAEL, Samuel G., Germantown, Maryland 20874 (US); VOSS, Ty C., Jefferson, Maryland 21755 (US); BALJINNYAM, Bolormaa, Rockville, Maryland 20852 (US); SIMEONOV, Anton, Bethesda, Maryland 20817 (US)
(74) Representative: Maiwald GmbH
(86) International application number: PCT/US2022/082171
(87) International publication number: WO 2023/122685

(56) References cited:
- WO-A1-2022/035743
- MARTINEZ NATALIA J. ET AL: "A widely-applicable high-throughput cellular thermal shift assay (CETSA) using split Nano Luciferase", SCIENTIFIC REPORTS, vol. 8, no. 1, 1 December 2018 (2018-12-01), pages 9472, XP055866053, Retrieved from the Internet <URL:https://www.nature.com/articles/s41598-018-27834-y.pdf> DOI: 10.1038/s41598-018-27834-y
- HENDERSON MARK J. ET AL: "High-throughput cellular thermal shift assays in research and drug discovery", SLAS DISCOVERY: ADVANCING LIFE SCIENCES R&D, vol. 25, no. 2, 2020, pages 137 - 147, XP055931705, ISSN: 2472-5552, Retrieved from the Internet <URL:https://www.sciencedirect.com/science/article/pii/S2472555222065315?via%3Dihub> DOI: 10.1177/2472555219877183
- ANONYMOUS: "The thermal shift assay: a powerful tool for analyzing proteins", 7 December 2021 (2021-12-07), XP093032859, Retrieved from the Internet <URL:https://bitesizebio.com/58311/thermal-shift-assay/> [retrieved on 20230320]
- SAMUEL ERROL L. G. ET AL: "Processing binding data using an open-source workflow", JOURNAL OF CHEMINFORMATICS, vol. 13, no. 1, 1 December 2021 (2021-12-01), XP093032865, Retrieved from the Internet <URL:https://jcheminf.biomedcentral.com/counter/pdf/10.1186/s13321-021-00577-1.pdf> DOI: 10.1186/s13321-021-00577-1
- SANCHEZ TINO W. ET AL: "Real-Time Cellular Thermal Shift Assay to Monitor Target Engagement", ACS CHEMICAL BIOLOGY, vol. 17, no. 9, 1 September 2022 (2022-09-01), pages 2471 - 2482, XP093032844, ISSN: 1554-8929, Retrieved from the Internet <URL:https://pubs.acs.org/doi/pdf/10.1021/acschembio.2c00334> DOI: 10.1021/acschembio.2c00334

## Description

### STATEMENT REGARDING FEDERALLY SPONSORED RESEARCH OR DEVELOPMENT

This invention was made with Government support by the National Institutes of Health, National Center for Advancing Translational Sciences. The Government has certain rights in this invention.

### BACKGROUND

The Cellular Thermal Shift Assay (CETSA) is an experimental procedure that enables, e.g., the assessment of drug-protein interaction by quantifying changes in the thermal stability of a protein upon ligand binding. However, the original CETSA protocol is significantly low-throughput, requires substantial optimization, and relies on time-consuming western blot detection of the target of interest. Further, traditional CETSA is limited to either a single temperature or a single compound concentration across a temperature range. In a modified CETSA method, the protein of interest is tagged with nanoLuciferase, a commonly-used luminescent protein, and, subsequently, the luciferase substrate is added to measure levels of the protein of interest. Then, the intact thermally-stable target of interest can be quantified by a luminescent signal. However, nanoLuciferase melts at too low a temperature to be used in a full CETSA temperature ramp.

Martinez et al., Scientific Reports, Vol. 8, No 1 (2018) discloses a high-throughput cellular thermal shift assay (CETSA) using split Nano Luciferase. Henderson et al., SLAS DISCOVERY: Advancing Life Sciences R&D, vol. 25, No. 2 (2022) discloses high-throughput cellular thermal shift assays in research and drug discoveries. Samuel et al., Journal of Cheminformatics, vol. 13, no. 1 (2021) discloses processing binding data using an open source workflow. Furthermore, URL:https:/ /bitesizebio.com/5831 1/thermal-shift-assay/ (2021) suggests thermal shift assays as a tool for analyzing proteins.

Accordingly, new methods and reporter molecules are needed which allow researchers to view thermal shift data via a luminescent signal, in real time, and across a full CETSA temperature ramp in order to generate, e.g., full aggregation profiles for multiple samples in parallel. In addition, improved methods and systems are needed for analyzing target engagement datasets from assays, including real time CETSA assays that produce kinetic readouts with dose response groups.

### BRIEF SUMMARY OF THE INVENTION

Aspects of the present disclosure include a system for analyzing data from a biological assay, wherein the biological assay comprises a biological system, and wherein the system for analyzing data identifies from one or more analytes those analytes demonstrating a desired activity in the biological system, wherein data from the biological assay is obtained using an analytical device, and wherein the data from the biological assay comprises results obtained at a plurality of experimental conditions, the experimental conditions varying with respect to tested values for a first independent variable and tested values for a second independent variable, wherein the tested values for the first independent variable collectively comprise a temperature gradient and wherein the second independent variable is the concentration of each of the one or more analytes present in each of the plurality of experimental conditions, and wherein the biological assay comprises Real Time Cellular Thermal Shift Assay (RT-CETSA), differential scanning fluorimetry, thermal shift analysis, intrinsic fluorescence differential scanning fluorimetry, or nano differential scanning fluorimetry (nanoDSF), the system comprising:
a memory storing instructions; and
one or more processors that, responsive to executing the instructions, are configured to:
   a) receive initial results from the analytical device;
   b) process the initial results to obtain processed results;
   c) fit the processed results for each concentration of a single analyte to a first model and a second model at each tested value of the first independent variable,
      wherein the first model is a linear null model with a slope of 0, and the second model is an alternative logarithmic model;
   d) determine a plurality of residual sum of squares (RSS) values for the first and second models at each tested value of the first independent variable;
   e) analyze the plurality of RSS values using a non-parametric goodness of fit test at each tested value of the first independent variable;
   f) responsive to determining for the single analyte that the second model is a better fit than the first model for at least one tested value of the first independent variable, identify the single analyte as having the desired activity in the biological system; and
   g) repeat steps (c)-(f) for each of the one or more analytes.

Aspects of the disclosure also include a method for analyzing data from a biological assay, wherein the biological assay comprises a biological system, and wherein the system for analyzing data identifies from one or more analytes those analytes demonstrating a desired activity in the biological system, wherein data from the biological assay is obtained using an analytical device, and wherein the data from the biological assay comprises results obtained at a plurality of experimental conditions, the experimental conditions varying with respect to tested values for a first independent variable and tested values for a second independent variable, wherein the tested values for the first independent variable collectively comprise a temperature gradient and wherein the second independent variable is the concentration of each of the one or more analytes present in each of the plurality of experimental conditions, and wherein the biological assay comprises Real Time Cellular Thermal Shift Assay (RT-CETSA), differential scanning fluorimetry, thermal shift analysis, intrinsic fluorescence differential scanning fluorimetry, or nano differential scanning fluorimetry (nanoDSF), the method comprising:
a) receiving initial results from the analytical device;
b) processing the initial results to obtain processed results;
c) fit the processed results for each concentration of a single analyte to a first model and a second model at each tested value of the first independent variable,
   wherein the first model is a linear null model with a slope of 0, and the second model is an alternative logarithmic model;
d) determining a plurality of residual sum of squares (RSS) values for the first and second models at each tested value of the first independent variable;
e) analyzing the plurality of RSS values using a non-parametric goodness of fit test at each tested value of the first independent variable;
f) responsive to determining for the single analyte that the second model is a better fit than the first model for at least one tested value of the first independent variable, identifying the single analyte as having the desired activity in the biological system; and
g) repeating steps (c)-(f) for each of the one or more analytes.

### BRIEF DESCRIPTION OF THE SEVERAL VIEWS OF THE DRAWING(S)

**Figure 1A** is a graph depicting the melting points for three different proteins by differential scanning fluorimetry. As can be seen in the graph, the LgBiT when incubated with a peptide comprising, consisting of, or consisting essentially of the HiBit fragment of NanoLuc with GlySer extensions had an increased melting point relative to the LgBiT fragment by itself and 156+Native peptide.
**Figure 1B** provides a visual depiction of an aspect of the reporter region of the disclosed protein constructs with ThermLuc as carboxy-terminal fusion (top) or amino-terminal fusion (bottom). The locations of Linker 1 and linker 2 are depicted.
**Figure 1C** depicts a comparison of the melting profile of 11s-86b fusion proteins with varying Gly-Ser linker lengths when expressed in HEK293T cells. When the size of the linker was increased to 3 or more GlySer repeats between the two fragments, the resultant fusion protein displayed minimal melting over the temperature ramp range commonly used in CETSA experiments.
**Figure 1D** depicts the amino acid sequence (SEQ ID NO: 23) of the reporter region designated "ThermLuc".
**Figure 1E** depicts a nucleotide sequence (SEQ ID NO: 28) encoding the reporter region designated "ThermLuc".
**Figure 1F** depicts a comparison of the melting profile of 11s-86b fusion proteins with varying Gly-Ser linker lengths when expressed in HEK293T cells and analyzed using the real-time analytical device according to the present disclosure. When the size of the linker was increased to 3 or greater Gly-Ser repeats between the two fragments, the resultant fusion protein displayed less melting than Nanoluciferase over the temperature ramp range commonly used in CETSA experiments.
**Figure 1G** depicts the melting profile of 11s-86b fusion proteins with varying gly-ser linker lengths when expressed in HEK293T cells and analyzed using the real-time analytical device Luminescence values are normalized to the 37 °C value for each fusion protein.
**Figure 2** visually depicts the configuration of an analytical device according to the present disclosure.
**Figure 3A** depicts a still image of a plate containing LDHA-ThermLuc transfected HEK293T cells during an RT-CETSA assay. This still image is representative of a single time point in the continuous, real-time, visualization of luminescence provided by RT-CETSA.
**Figure 3B** depicts the results of an experiment utilizing HEK293T cells wherein the target protein of interest is LDHA fused to either ThermLuc or Nanoluciferase, where thermal shift is only detectable for the ThermLuc fusion.
**Figure 3C** depicts results indicating that some compounds may show binding in the DSF assay with purified protein, but not CETSA, because they do not bind the target in cells, for instance because they lack membrane permeability.
**Figure 3D** depicts data regarding thermal shifts across a multi-well plate.
**Figure 3E** depicts data regarding thermal shifts across a multi-well plate.
**Figure 3F** depicts data regarding thermal shifts across a multi-well plate.
**Figure 3G** presents a visual depiction of data indicating that LDHA inhibitors show a dose dependent shift.
**Figure 3H** depicts the RT-CETSA melt profile of eight examples of ThermLuc fused to target proteins of interest.
**Figure 3I** depicts target engagement in RT-CETSA.
**Figure 3J** depicts target engagement in RT-CETSA.
**Figure 3K** depicts target engagement in RT-CETSA.
**Figure 4A** provides a graph indicating that the melting of target proteins (exemplified by LDHA-ThermLuc) occurs within seconds of the application of heat. Accordingly, RT-CETSA allows for a real-time read out of target melting and small molecule engagement with temporal resolution that cannot be obtained with other CETSA techniques.
**Figure 4B** depicts the thermal aggregation profile of immunotherapeutic targets obtained with traditional CETSA (as compared to RT-CETSA in Fig. 4C).
**Figure 4C** depicts the thermal aggregation profile of immunotherapeutic targets obtained with RT-CETSA (as compared to traditional CETSA in Fig. 4B).
**Figure 5** depicts an example of a multi-target readout obtained by RT-CETSA.
**Figure 6** provides a visual depiction of ThermLuc being inserted into a pcDNA3.1 vector with proper In-Fusion (Takara Bio) homologous sequences, containing a BamHI restriction site (encoding Gly-Ser) at the junction between ThermLuc and the target.
**Figure 7A** is a schematic diagram illustrating dihydrofolate reductase (DHFR)-ThermLuc constructs that were prepared with various first peptide linker region sequences between the target of interest (i.e. DHFR) and the reporter molecule (i.e. ThermLuc) (SEQ ID NOS: 1-17).
**Figure 7B** is a graph depicting the cellular thermal melt profiles of DHFR fusion proteins comprising NanoLuc and ThermLuc.
**Figure 7C** is a graph depicting the thermal stability of DHFR-ThermLuc fusions when separated by various first peptide linker sequences.
**Figure 8** is a flow chart depicting a method **800** for applying the disclosed analytical methods.
**Figure 9** is a diagram depicting a computer system **900** for use in the disclosed analytical methods.
**Figure 10** is a diagram depicting the RT-CETSA workflow
**Figure 11A** depicts a concentration-response baseline-corrected thermal unfolding curves from an RT-CETSA assay.
**Figure 11B** is a pair of graphs depicting null and alternate models used to fit the response curve for LDHAi NCGC00372530-01 at 64 °C in the analysis of data from an RT-CETSA assay.
**Figure 11C** is a graph depicting the residual sum of squares (RSS) values for the null and alternate models plotted against the temperature ramp of a representative RT-CETSA experiment.
**Figure 11D** is a graph plotting the fraction of unfolded protein vs. the concentration of LDHAᵢ NCGC00372530-01.
**Figure 11E** is a graph depicting the single parameter T_{agg} and AUC (of fraction unfolded) derived EC₅₀ values for LDHAᵢ NCGC00372530-01 concentration-response data.
**Figure 12A** is a diagram depicting Log EC₅₀ values for a plate of LDHAi inhibitors (n=3) when analyzed with RT-CETSA methods and SplitLuc CETSA. Darker shading indicates lower EC₅₀ values (i.e., more potent activity).
**Figure 12B** is a diagram depicting Spearman coefficients showing high correlation with single parameter analysis of RT-CETSA data and SplitLuc CETSA methods.
**Figure 12C** is three boxplot distributions of positive and negative controls using LDHA-ThermLuc that are used to determine the Z' statistic and signal window using Tagg, AUC, and NPARC methods of analysis. Solid lines represent the means of each group, and dashed lines represent the ±3*SD for each control group.
**Figure 12D** is a graph depicting the testing of the minimum significant ratio (MSR) and related parameters further characterizing the high reproducibility of potency estimates from the RT-CETSA method. The mean ratio (MR) is shown as a solid blue line, Limits of Agreement (LsA) in dashed lines, and ratio limits (RL) in dashed lines.

### DETAILED DESCRIPTION

### Protein Constructs

Disclosed but not claimed are protein constructs comprising, consisting of, or consisting essentially of a target protein of interest, a first peptide linker, and a reporter region.

In aspects, the protein construct comprises, consists of, or consists essentially of, from N-terminus to C-terminus, the target protein of interest, the first peptide linker, and the reporter region. In other aspects, the protein construct comprises, consists of, or consists essentially of, from N-terminus to C-terminus, the reporter region, the first peptide linker, and the target protein of interest.

The target protein of interest may be any protein. In some aspects the target protein of interest may be a full-length protein. The target protein of interest may be, for example any protein in the proteome of a mammalian cell. The target protein of interest may also be a protein fragment. The target protein of interest may also be a complex of multiple peptides or proteins. Examples of suitable target proteins of interest include potential pharmaceutical targets, proteins involved in the inflammatory process, proteins involved in regulating the cell cycle, proteins involved in cancer cell proliferation or cancer cell metabolism, proteins involved in disease or other pathologies, and proteins with unknown functions.

Examples of specific target proteins of interest include, without limitation, lactate dehydrogenase A ("LDHA"), mammalian tyrosine-protein kinase ABL1 ("c-Abl"), B-lymphocyte antigen CD19 ("CD19"), B-lymphocyte antigen CD20 ("CD20"), programmed cell death protein 1 ("PD1"), cytotoxic T-lymphocyte-associated protein 4 ("CTLA4"), nerve growth factor ("NGF"), dihydrofolate reductase ("DHFR") and proprotein convertase subtilisin/kexin type 9 ("PCSK9"). In aspects, the target protein of interest may be a protein that has been modified relative to wild-type. Examples of modified proteins include, for example, proteins in which one or more point mutations have been introduced.

In aspects, the target protein of interest is LDHA, DHFR, c-Abl, CD19, CD20, PD1, CTLA 4, NGF or PCSK9.

The first peptide linker may be any suitable sequence of amino acids. In aspects, the sequence of the first peptide linker comprises, consists of, or consists essentially of a polypeptide having greater than 80% identity with any one of SEQ ID NOS: 1-17. In aspects, the sequence of the first peptide linker comprises, consists of, or consists essentially of a polypeptide having greater than 85% identity with any one of SEQ ID NOS: 1-17. In aspects, the sequence of the first peptide linker comprises, consists of, or consists essentially of a polypeptide having greater than 90% identity with an one of SEQ ID NOS: 1-17. In aspects, the sequence of the first peptide linker comprises, consists of, or consists essentially of a polypeptide having greater than 95% identity with any one of SEQ ID NOS: 1-17. In aspects, the sequence of the first peptide linker comprises, consists of, or consists essentially of any one of SEQ ID NOS: 1-17.

The reporter region comprises, consists of, or consists essentially of, from N-terminus to C-terminus, a LgBiT fragment, a second peptide linker, and a HiBiT fragment.

In aspects, the LgBiT fragment is a fragment of nanoluciferase ("NanoLuc"), which is also referred to herein as "11s". 11s has the amino acid sequence of SEQ ID NO: 18. In other aspects, the LgBiT fragment comprises, consists of, or consists essentially of a polypeptide having at least 80% identity with SEQ ID NO: 18. In other aspects, the LgBiT fragment comprises, consists of, or consists essentially of a polypeptide having at least 85% identity with SEQ ID NO: 18. In other aspects, the LgBiT fragment comprises, consists of, or consists essentially of a polypeptide having at least 90% identity with SEQ ID NO: 18. In other aspects, the LgBiT fragment comprises, consists of, or consists essentially of a polypeptide having at least 95% identity with SEQ ID NO: 18.

The second peptide linker comprises, consists of, or consists essentially of any suitable sequence of amino acids. In some aspects, the second peptide linker comprises, consists of, or consists essentially of one or more glycine-serine ("GlySer") repeats. In some aspects the second peptide linker comprises, consists of, or consists essentially of one GlySer repeat, two GlySer repeats, three GlySer repeats, four GlySer repeats, five GlySer repeats or six GlySer repeats. In other aspects the second peptide linker comprises, consists of, or consists essentially of greater than 6 GlySer repeats. In aspects, the second peptide linker comprises, consists of, or consists essentially of seven GlySer repeats, eight GlySer repeats, nine GlySer repeats, ten GlySer repeats, eleven GlySer repeats, twelve GlySer repeats, thirteen GlySer repeats, fourteen GlySer repeats or fifteen GlySer repeats. In one aspect, the second peptide linker is 6 GlySer repeats (SEQ ID NO: 19).

In aspects, the HiBiT fragment is a fragment of NanoLuc having the amino acid sequence of SEQ ID NO: 20. In other aspects, the HiBiT fragment comprises, consists of, or consists essentially of a polypeptide having at least 80% identity with SEQ ID NO: 20. In other aspects, the HiBiT fragment comprises, consists of, or consists essentially of a polypeptide having at least 85% identity with SEQ ID NO: 20. In other aspects, the HiBiT fragment comprises, consists of, or consists essentially of a polypeptide having at least 90% identity with SEQ ID NO: 20. In other aspects, the HiBiT fragment comprises, consists of, or consists essentially of a polypeptide having at least 95% identity with SEQ ID NO: 20.

In aspects, the HiBiT fragment additionally comprises, consists of, or consists essentially of one or more GlySer extensions. For example, in an aspect a GlySer extension is present at the C-terminus of the HiBit fragment. In one such aspect the HiBiT fragment comprises, consists of, or consists essentially of SEQ ID NO: 20 with GlySer at the C-terminus (SEQ ID NO: 21). In other aspects that HiBiT fragment comprises, consists of, or consists essentially of a polypeptide having at least 80% identity with SEQ ID NO: 21. In other aspects the HiBiT fragment comprises, consists of, or consists essentially of a polypeptide having at least 85% identity with SEQ ID NO: 21. In other aspects that HiBiT fragment comprises, consists of, or consists essentially of a polypeptide having at least 90% identity with SEQ ID NO: 21. In other aspects that HiBiT fragment comprises, consists of, or consists essentially of a polypeptide having at least 95% identity with SEQ ID NO: 21.

In other aspects, the HiBiT fragment comprises, consists of, or consists essentially of a GlySer extension at both the N-Terminus and the C-terminus. In one such aspect, the HiBiT fragment comprises, consists of, or consists essentially of SEQ ID NO: 20 with GlySer at the N-terminus and GlySer at the C-terminus. This peptide sequence is referred to as "86b" (SEQ ID NO: 22). In other aspects that HiBiT fragment comprises, consists of, or consists essentially of a polypeptide having at least 80% identity with SEQ ID NO: 22. In other aspects that HiBiT fragment comprises, consists of, or consists essentially of a polypeptide having at least 85% identity with SEQ ID NO: 22. In other aspects that HiBiT fragment comprises, consists of, or consists essentially of a polypeptide having at least 90% identity with SEQ ID NO: 22. In other aspects that HiBiT fragment comprises, consists of, or consists essentially of a polypeptide having at least 95% identity with SEQ ID NO: 22.

In aspects, the reporter region is a polypeptide having the amino acid sequence of SEQ ID NO: 23 (referred to as "ThermLuc"). In other aspects, the reporter region comprises, consists of, or consists essentially of a polypeptide having at least 80% identity with SEQ ID NO: 23. In other aspects, the reporter region comprises, consists of, or consists essentially of a polypeptide having at least 85% identity with SEQ ID NO: 23. In other aspects, the reporter region comprises, consists of, or consists essentially of a polypeptide having at least 90% identity with SEQ ID NO: 23. In other aspects, the reporter region comprises, consists of, or consists essentially of a polypeptide having at least 95% identity with SEQ ID NO: 23.

### Biological Vector

Disclosed but not claimed is a biological vector encoding the protein construct.

In an aspect of the disclosure, the vector is a recombinant expression vector. For purposes herein, the term "recombinant expression vector" means a genetically-modified oligonucleotide or polynucleotide construct that permits the expression of an mRNA, protein, polypeptide, or peptide by a host cell, when the construct comprises, consists of, or consists essentially of a nucleotide sequence encoding the mRNA, protein, polypeptide, or peptide, and the vector is contacted with the cell under conditions sufficient to have the mRNA, protein, polypeptide, or peptide expressed within the cell. The disclosed vectors are not naturally-occurring as a whole. However, parts of the vectors can be naturally-occurring. The recombinant expression vectors can comprise any type of nucleotides, including, but not limited to DNA and RNA, which can be single-stranded or double-stranded, synthesized or obtained in part from natural sources, and which can contain natural, non-natural or altered nucleotides. The recombinant expression vectors can comprise naturally-occurring, non-naturally-occurring internucleotide linkages, or both types of linkages. Preferably, the non-naturally occurring or altered nucleotides or internucleotide linkages does not hinder the transcription or replication of the vector.

The recombinant expression vectors can be prepared using standard recombinant DNA techniques. Constructs of expression vectors, which are circular or linear, can be prepared to contain a replication system functional in a prokaryotic or eukaryotic host cell. Replication systems can be derived, e.g., from ColEl, 2 µ plasmid, λ, SV40, bovine papilloma virus, and the like.

The recombinant expression vector can include one or more marker genes, which allow for selection of transformed or transfected hosts. Marker genes include biocide resistance, e.g., resistance to antibiotics, heavy metals, etc., complementation in an auxotrophic host to provide prototrophy, and the like. Suitable marker genes for the disclosed expression vectors include, for instance, neomycin/G418 resistance genes, hygromycin resistance genes, histidinol resistance genes, tetracycline resistance genes, and ampicillin resistance genes.

The vector may further comprise regulatory sequences that are operably linked to the nucleotide sequence encoding the protein constructs which permits one or more of the transcription, translation, and expression protein constructs in a cell transfected with the vector or infected with a virus that comprises, consists of, or consists essentially of the vector. As used herein, "operably linked" sequences include both regulatory sequences that are contiguous with the nucleotide sequence encoding the protein construct and regulatory sequences that act in trans or at a distance to control the nucleotide sequence encoding the protein construct.

The regulatory sequences may include appropriate transcription initiation, termination, promoter and enhancer sequences; RNA processing signals such as splicing and polyadenylation (polyA) signal sequences; sequences that stabilize cytoplasmic mRNA; sequences that enhance translation efficiency (i.e., Kozak consensus sequence); sequences that enhance protein stability.

In aspects, the biological vector comprises, consists of, or consists essentially of a promotor that drives expression of the protein construct. The promoter may be any promoter suitable for expressing the protein construct in a target cell, e.g., a mammalian cell. The promoter may be inducible or constitutive. In an aspect of the disclosure, the promoter is suitable for expressing the protein construct in a particular cell type. In this regard, the promoter may be cell-specific.

In some aspects, the vector is a pcDNA3.1 vector.

In some aspects, the vector is a viral vector. Examples of suitable viral vectors include retroviral vectors, lentiviral vectors, adenoviral vectors, adeno-associated viral (AAV) vectors.

In some aspects, the biological vector is prepared by inserting the sequence encoding the protein construct into a universal acceptor plasmid. For example, a nucleotide sequence encoding the protein construct may be inserted into a pcDNA3.1 vector with proper In-Fusion consensus sequences.

In aspects, the biological vector comprises, consists of, or consists essentially of nucleotides encoding the LgBiT fragment of the protein construct, for example SEQ ID NO: 24. In other aspects, the biological vector comprises, consists of, or consists essentially of a nucleotide sequence having greater than 80% identity to SEQ ID NO: 24. In other aspects the biological vector comprises, consists of, or consists essentially of a nucleotide sequence having greater than 85% identity to SEQ ID NO: 24. In other aspects the biological vector comprises, consists of, or consists essentially of a nucleotide sequence having greater than 90% identity to SEQ ID NO: 24. In other aspects the biological vector comprises, consists of, or consists essentially of a nucleotide sequence having greater than 95% identity to SEQ ID NO: 24.

In aspects, the biological vector comprises, consists of, or consists essentially of nucleotides encoding the HiBiT fragment of the protein construct, for example, SEQ ID NOS: 25, 26 or 27. In aspects the biological vector comprises, consists of, or consists essentially of a nucleotide sequence having greater than 80% identity to SEQ ID NO: 25. In other aspects the biological vector comprises, consists of, or consists essentially of a nucleotide sequence having greater than 85% identity to SEQ ID NO: 25. In other aspects the biological vector comprises, consists of, or consists essentially of a nucleotide sequence having greater than 90% identity to SEQ ID NO: 25. In other aspects the biological vector comprises, consists of, or consists essentially of a nucleotide sequence having greater than 95% identity to SEQ ID NO: 25. In aspects, the biological vector comprises, consists of, or consists essentially of the nucleotide sequence SEQ ID NO: 26. In other aspects the biological vector comprises, consists of, or consists essentially of a nucleotide sequence having greater than 80% identity to SEQ ID NO: 26. In other aspects the biological vector comprises, consists of, or consists essentially of a nucleotide sequence having greater than 85% identity to SEQ ID NO: 26. In other aspects the biological vector comprises, consists of, or consists essentially of a nucleotide sequence having greater than 90% identity to SEQ ID NO: 26. In other aspects the biological vector comprises, consists of, or consists essentially of a nucleotide sequence having greater than 95% identity to SEQ ID NO: 26. In aspects, the biological vector comprises, consists of, or consists essentially of the nucleotide sequence SEQ ID NO: 27. In other aspects the biological vector comprises, consists of, or consists essentially of a nucleotide sequence having greater than 80% identity to SEQ ID NO: 27. In other aspects the biological vector comprises, consists of, or consists essentially of a nucleotide sequence having greater than 85% identity to SEQ ID NO: 27. In other aspects the biological vector comprises, consists of, or consists essentially of a nucleotide sequence having greater than 90% identity to SEQ ID NO: 27. In other aspects the biological vector comprises, consists of, or consists essentially of a nucleotide sequence having greater than 95% identity to SEQ ID NO: 27.

In aspects, the biological vector comprises, consists of, or consists essentially of a nucleotide sequence encoding ThermLuc, for example SEQ ID NO: 28. In aspects the biological vector comprises, consists of, or consists essentially of a nucleotide sequence having greater than 80% identity to SEQ ID NO: 28. In other aspects the biological vector comprises, consists of, or consists essentially of a nucleotide sequence having greater than 85% identity to SEQ ID NO: 28. In other aspects the biological vector comprises, consists of, or consists essentially of a nucleotide sequence having greater than 90% identity to SEQ ID NO: 28. In other aspects the biological vector comprises, consists of, or consists essentially of a nucleotide sequence having greater than 95% identity to SEQ ID NO: 28.

In aspects, the biological vector comprises, consists of, or consists essentially of a nucleotide sequence encoding a second peptide linker. In aspects, the nucleotide sequence encodes a second peptide linker comprising Gly-Ser repeats, for example six Gly-Ser repeats. In aspects, the biological vector comprises, consists of, or consists essentially of a nucleotide sequence comprising SEQ ID NO: 29.

### RT-CETSA Method

Disclosed but not claimed is a method for utilizing the disclosed protein constructs to test samples, wherein the samples comprise living, intact cells. The method comprises, consists of, or consists essentially of:
a) transfecting the cells with a biological vector encoding the protein construct under conditions suitable to allow the expression of the protein construct within the cells;
c) exposing the expressed protein construct to a photon generating substrate; and
d) exposing the cells to an increasing temperature gradient while detecting the change in luminescence of the sample in real time.

In aspects, the method is a Real Time Cellular Thermal Shift Assay, which allows researchers to view thermal shift data in real time.

The cells for use in the disclosed methods may be any suitable cells. For example, the cells may be mammalian cells. In some aspects, the cells are human. Examples of suitable mammalian cell lines include the Chinese hamster ovary (CHO), COS, and human cell lines such as HEK and HeLa. In some aspects, the cells are HEK293T cells. The cells may be cultured in any suitable media according to methods known in the art. For example, HEK293T cells may be cultured in DMEM, 4.5 g/L glucose (Invitrogen) with 10% fetal bovine serum (FBS), 6 mM L-glutamine, 1 mM sodium pyruvate, 50 U/mL penicillin, and 50 µg/mL streptomycin. In an aspect, the cells may be in placed into suspension. In aspects, the suspension comprising, consisting of, or consisting essentially of the cells may be transferred to wells in multi-well plates.

In aspects, the biological vector is transfected into the cells. A number of transfection techniques are generally known in the art (see, e.g., Graham et al., Virology, 52: 456-467 (1973); Sambrook et al. Molecular Cloning: A Laboratory Manual (Third Edition) (Cold Spring Harbor Laboratory Press, 2000); Davis et al., Basic Methods in Molecular Biology, Elsevier (1986); and Chu et al., Gene, 13: 97 (1981). Transfection methods include calcium phosphate coprecipitation, direct micro injection into cultured cells (see, e.g., Capecchi, Cell, 22: 479-488 (1980)), electroporation (see, e.g., Shigekawa et al., BioTechniques, 6: 742-751 (1988)), liposome mediated gene transfer (see, e.g., Mannino et al., BioTechniques, 6: 682-690 (1988)), lipid mediated transduction (see, e.g., Felgner et al., Proc. Natl. Acad. Sci. USA, 84: 7413-7417 (1987)), and nucleic acid delivery using high velocity microprojectiles (see, e.g., Klein et al., Nature, 327: 70-73 (1987)). In some aspects, the vector is transiently transfected into the cells. In other aspects, stable transfection is utilized. Transfection, as used herein, also refers to viral transduction in aspects wherein the biological vector is a viral vector.

In an aspect, the expressed protein construct is contacted with a photon generating substrate. An example of a photon generating substrate is furimazine. This can be achieved via any appropriate laboratory technique. For example, where the cells are in suspension, a suitable amount of furimazine may be added to the suspension.

Aspects of the disclosure comprise exposing the cells to an increasing temperature gradient. "Increasing temperature gradient", as used herein, refers to a temperature that increases from a starting temperature to a final temperature over time. Different starting temperatures may be used, for example about 20 °C, about 30 °C, about 40 °C. In many aspects the starting temperature will be between 30 °C and 37 °C. In some aspects, the starting temperature will be room temperature. Different final temperatures may also be used, for example about 90 °C, 80 °C, 70 °C, 60 °C, or 50 °C. Any suitable combination of starting and final temperatures may be used. The increasing temperature gradient may have a starting temperature of, for example, 20 °C and an ending temperature of about 80 °C. Alternatively, the increasing temperature gradient may have a have a starting temperature of about 30 °C and an ending temperature of about 70 °C. As another example, the increasing temperature gradient may have a starting temperature of about 40 °C and an ending temperature of about 60 °C. Any suitable rate of temperature increase may be used in the increasing temperature gradient. In one aspect the rate is about 0.2 °C per second. Alternatively, faster or slower rates of increase may be used.

Aspects of the method further comprise contacting the protein construct with one or more analytes or additional test molecules. In such aspects, the RT-CETSA assay may be used to detect binding (or lack thereof) between the target protein of interest and the test analyte in the cells. The analyte may be a small molecule. In aspects, the analyte binds to the target protein of interest. Examples of suitable small molecules include, without limitation, potential drug candidates, ligands known to bind to the target protein(s) of interest, known inhibitors of the target protein(s) of interest, and molecules with unknown biological activity.

The analyte(s) may also be larger molecules such as, for example, proteins and antibodies. In some aspects, the analyte is a monoclonal antibody. Methods for obtaining and preparing monoclonal antibodies are known to those skilled in the art.

Aspects of the disclosure also allow for the parallel testing of multiple samples with different temperatures of aggregation in a high-throughput environment. The samples may contain different target proteins of interest and/or different analytes from one another. Such samples may be assayed together in, for example, a multi-well plate.

### Analytical Device

Disclosed but not claimed is an analytical device; wherein the analytical device is capable of simultaneously collecting real time luminescence data during a temperature hold or ramp for multiple samples. In an aspect, the device comprises, consists of, or consists essentially of: (a) a thermal cycler block adapted to receive a multi-well plate comprising, consisting of, or consisting essentially of the multiple samples; (b) a detection device capable of detecting luminescence; and (c) a thermal top-heat assembly adapted to maintain even heating across the top of the multi well plate and to allow a luminescent signal to pass through to the detection device. The detection device is positioned such that it can detect changing luminescence in the multiple samples in real time.

In aspects, the analytical device comprises, consists of, or consists essentially of a thermal cycler. A thermal cycler, as disclosed herein, is a laboratory apparatus typically used to amplify segments of DNA via the polymerase chain reaction. A suitable thermal cycler is capable of applying heat to the samples being tested to achieve an increasing temperature gradient consistent across the multiwall plate. Thermal cyclers according to the present disclosure comprise, consist of, or consist essentially of a thermal block adapted to receive samples. In an aspect, the block is adapted to receive one or more multi-well sample plates. Aspects of the disclosure include the modification of commercially available thermal cyclers by, e.g., removing excitation and emissions filters and/or exchanging detection devices to increase sensitivity to luminescence.

Suitable detection devices for detecting luminescence are known to those skilled in the art. For example, in an aspect the detection device is a sensitive CCD or CMOS sensor. The sensor may be cooled, for example, water cooled. An example of a suitable CCD camera is the ORCA II (Hamamatsu).

In aspects, the thermal cycler is adapted to receive multi well plates. Such plates include 96-well plates, 384-well plates, and 1536-well plates, all of which are readily available and familiar to those skilled in the art.

Aspects also include a thermal top-heat assembly that is positioned above the multi-well plate and is adapted to ensure event heating across the plate without impeding the luminescent signal.

### Methods for Data Analysis

The disclosure also provides methods for analyzing data obtained from a biological assay comprising Real Time Cellular Thermal Shift Assay (RT-CETSA), differential scanning fluorimetry, thermal shift analysis, intrinsic fluorescence differential scanning fluorimetry, or nano differential scanning fluorimetry (nanoDSF). For example, the disclosed method can analyze a wide range of thermal shift data that contains concentration-response groups. In certain aspects, the biological assay for use in combination with the disclosed methods for data analysis is an RT-CETSA assay as disclosed herein.

In certain aspects of the present disclosure, the biological assay is a high throughput assay in which multiple samples are tested in parallel, simultaneously, and/or nearly simultaneously. The samples may contain different experimental conditions as well as, e.g., in the context of RT-CETSA, different target proteins of interest and/or different additional analytes from one another. Such samples may be assayed together in, for example, a multi-well plate.

In aspects of the disclosure, the biological assay generating the data to be analyzed comprises a biological system. "Biological system", as used herein, refers to any composition of matter or set of conditions that comprises models or approximates a biological process or processes. Such systems may include various proteins, peptides and nucleic acids. In aspects such proteins, peptides and/or nucleic acids may be relevant to disease states in e.g., animals or humans. In aspects of the disclosure, the system may comprise living cells maintained under suitable conditions, such as, e.g., the living cells in an RT-CETSA assay.

In aspects of the disclosure wherein the biological assay generating the data to be analyzed is RT-CETSA, the biological system may comprise samples of living, intact cells. As disclosed herein, the cells may be transfected with a biological vector encoding the disclosed protein constructs comprising the target protein of interest, such that the protein constructs are expressed in the cell.

In aspects, the disclosed methods comprise analyzing data with respect to one or more analytes. In the context of the present disclosure, "analyte" and refers to any compound, composition or substance being tested for activity in the relevant biological system. Suitable analytes include small molecules such as potential drug candidates, ligands known to bind to the target protein(s) of interest, known inhibitors of the target protein(s) of interest, and molecules with unknown biological activity. The one or more analytes may also comprise larger molecules such as, for example, proteins and antibodies. In some aspects, the additional test molecule is a monoclonal antibody.

In aspects, the disclosed methods for data analysis determine whether the analyte has desired activity in the biological system. The "desired activity" may be any property of the analyte that impacts or alters the functioning of the biological system, as informed by the underlying biology of the system. Examples include wherein the analyte is a ligand that binds to a target protein, wherein the analyte facilitates the targeting of particular cells, wherein the analyte inhibits the functioning of a target enzyme, and wherein the analyte alters the expression of a target gene. Those skilled in the art will readily understand the desired activity will be context dependent based on the nature of the biological assay, the biological system and the methods used for data collection. Those skilled in the art will further understand that whether an activity is "desired" will depend on the nature of the system under investigation. For example, a small molecule ligand's activity inhibiting a protein may be desirable in some contexts and undesirable in others. Those skilled in the art will be readily able to identify the appropriate activity for the purposes of the disclosed analytical methods based on biological and technological context.

In aspects of the disclosure where the biological assay generating data for analysis is RT-CETSA, the RT-CETSA assay may be used as a way of elucidating binding (or lack thereof) between the target protein of interest and the analyte in living cells. Accordingly, the "desired activity" may be the ability of the analyte to bind to a target protein. Such binding may result in a thermal stabilizing effect that can be detected by analyzing the data from an RT-CETSA assay according to the disclosed methods.

In aspects of the present disclosure, results are collected under one or more experimental conditions. In aspects, the experimental conditions in a given assay may vary with respect to one or more independent variables. Such variables may describe any suitable aspect of the experimental conditions. Independent variables refer to those conditions that may be controlled or manipulated by investigators in the biological assay. For example, independent variables may comprise elapsed time or temperature. Independent variables may also comprise, e.g., concentration of analyte or denaturant, pressure, or other experimentally modified condition. The tested values for the first independent variable collectively comprise a temperature gradient and the second independent variable is the concentration of each of the one or more analytes present in each of the plurality of experimental conditions. Values for other independent variables may similarly be selected to comprise a gradient analogous to the temperature gradients described herein.

The experimental conditions vary with respect to at least two independent variables, one of which is the concentration(s) of the one or more analytes, e.g., the second of the two variables may be analyte concentration. In aspects, data may be collected for multiple samples each containing a different concentration of a given analyte. In aspects where one of the independent variables is analyte concentration, the data may provide a dose response curve. The data may further comprise separate dose response curves for each tested value of the other independent variable. Such results may be used to investigate dose-response relationships using the disclosed analytical methods.

In aspects of the disclosure wherein the biological assay generating data to be analyzed is RT-CETSA, samples containing a range of concentrations of the test analyte may be exposed to a temperature gradient. Accordingly, in the context of an RT-CETSA assay, the experimental conditions may vary with respect to temperature (first independent variable) and the concentration of the test analyte (second independent variable). In such aspects, the results may comprise a dose response curve at each measured temperature. The resultant RT-CETSA data may be used, via the disclosed analytical methods, to determine whether a concentration dependent thermal stabilizing effect is present, thereby elucidating the binding of the analyte to the target protein of interest, via analytical methods as disclosed herein.

In aspects, data is collected via an analytical device prior to analysis. Those skilled in the art will be familiar with various suitable analytical devices capable of collecting and transmitting data readouts pertaining to various biological assays.

In aspects, the analytical device collects data for multiple sets of experimental conditions. In aspects, multiple samples that differ with respect to the concentration of an analyte may be tested as well as with respect to one or more other independent variables. Such experimental design allows for the collection of data reflecting a range of analyte concentration, which facilitates the determination of dose/response relationships via the disclosed methods. In aspects, different experimental conditions may be present in each well of a multiwell plate.

In aspects of the present disclosure, the analytical device may be a device configured to perform RT-CETSA assays as disclosed herein. In such aspects, the analytical device detects the change in luminescence of the sample(s) in real time over the course of the assay and may then transmit the corresponding data to an appropriate system (e.g., a computer) for further analysis. Aspects of the disclosure involving RT-CETSA allow for the parallel testing of multiple samples with target proteins of interest having different temperatures of aggregation in a high-throughput environment. The samples may contain different sets of conditions with respect to the protein constructs, the target proteins of interest and/or may contain different analytes, wherein each analyte is tested at a range of concentrations. Such samples may be assayed together in, for example, a multi-well plate with different conditions in each of the various wells.

In aspects of the present disclosure, "initial results" refers to the relevant data collected directly by an analytical device as part of the biological assay, e.g., the initial results may be the readout of the assay as measured or collected by the analytical device. In aspects, the analytical device transmits the collected initial results to a suitable system, for example a computer, via a suitable network or connection for analysis.

In aspects, the data received from the analytical device undergoes various forms of processing to generate processed results for further analysis. In this regard, initial results may be further processed in any manner necessary to render the results in a suitable form for analysis via the disclosed methods. Such processing may include formatting, organizing, converting and normalizing the results. In certain aspects, baseline correction is applied to the initial results. Methods for processing data will be well within the abilities of those skilled in the art.

For example, in aspects of the present disclosure wherein the biological assay is RT-CETSA, the results are initially in the form of image files, for example TIFF or JPEG files. The image files may correspond to each temperature step detected in the assay and depict the detected luminescence of the various wells in, e.g., a 384 well plate. In such aspects, processing may involve organizing the images by assigning well addresses and by splitting the data into smaller sets, for example multiple 96-well sets. Additional processing may be carried out by suitable software programs and scripts, as will be familiar to those skilled in the art. For example, initial results may be processed by MOLTENPROT (CSSB, Hamburg, Germany), which can be used to generate baseline corrected data.

The term "model" as it is used herein refers to a mathematical operation that seeks to characterize the relationship, if any, between data points in a given set of data. In aspects of the present disclosure, models are applied to, e.g., dose response curves. Generally, models may generally include linear discriminant analysis models, support vector machine classification algorithms, regression feature elimination models, predictive analysis of microarray models, and logistic models. Such models are well known to those skilled in the art, and those skilled in the art will be readily able to identify suitable models for a given application of the disclosed methods.

Aspects of the disclosure comprise the use of a first model, wherein the model is a linear model. In aspects, this model is constrained to slope = 0, and may be referred to as a null model. The null model assumes that the analyte does not have the "desired activity" in the biological system. For example, in the context of RT-CETSA, the null model assumes that there is no thermal stabilizing effect caused by the analyte binding to the target of interest.

In aspects, the disclosed methods further comprise using a second model. In aspects, the model is suitable for calculating the point of half-maximal response in a way that represents biological responses. The second model may comprise, for example, biphasic curves, Weibull model fits and/or log logistic models. In aspects, the second model is a log-logistic model. As will be familiar to those skilled in the art, log-logistic models are frequently used dose-response models for fitting bioassay data, including, e.g., in dose titration applications where data is linear for a short portion, and then approaches plateaus near the minimum and maximum doses. Log-logistic models according to the present disclosure may have 3-5 parameters. In aspects, the log-logistic model has four parameters, denoting the upper and lower asymptotes, inflection point, and Hill coefficient. The use of such models is known to those skilled in the art, see, e.g., Ritz et al., PLoS One, 2015:10(12):e0146021

In aspects of the disclosure, the two models are applied to the data for each experimental condition. In aspects, the residual sum of squares (RSS) values are then calculated for both of the models for each experimental condition, e.g., for each value of the first independent variable and second independent variable. Those skilled in the art will be familiar with calculating RSS values.

According to aspects of the disclosure, this means that for a given value of the first independent variable, the two models are applied to processed results obtained for all values of the second independent variable (i.e., for each analyte concentration), and RSS is then calculated at the given value of the first independent variable. This process is then iterated for all tested values of the first independent variable. For example, in the context of an RT-CETSA assay, for any given temperature, both models are applied to the processed results (comprising collected luminescence data as described above) for each tested concentration of the analyte, in other words, the two models are applied to the dose response curve at the given temperature. The RSS value is then calculated at the given temperature. The process is then repeated at each temperature at which data was collected.

The RSS values for both models are then compared at each value for the first independent variable using a non-parametric goodness-of-fit test, for example a Mann-Whitney U non-parametric test. The Mann-Whitney U non-parametric test will be familiar to those skilled in the art.

According to aspects of the disclosure, the analyte is determined to have the desired activity in the biological system if the Mann-Whitney U test indicates that the second model is a better fit at a given value of the first independent variable than the first (null) model at one or more of the tested values of the first independent variable. For example, in the analysis of RT-CETSA data, an analyte will be identified as having the desired activity if, at one or more temperatures, the log logistic model is a better fit than the null model based on the Mann-Whitney U test. In aspects the log-logistic model may be a better fit across multiple temperature points along the gradient.

In aspects, the half maximal effective concentration (EC₅₀) for an analyte may be calculated by plotting the processed RSS values from each model fit at each value of the first independent variable (e.g. temperature), and then fitting a 4-parameter log logistic model using the fraction unfolded values (for each concentration of analyte in the dose-response) at the independent variable value where the amount of RSS difference between the first and second models is greatest.

### Systems for Data Analysis

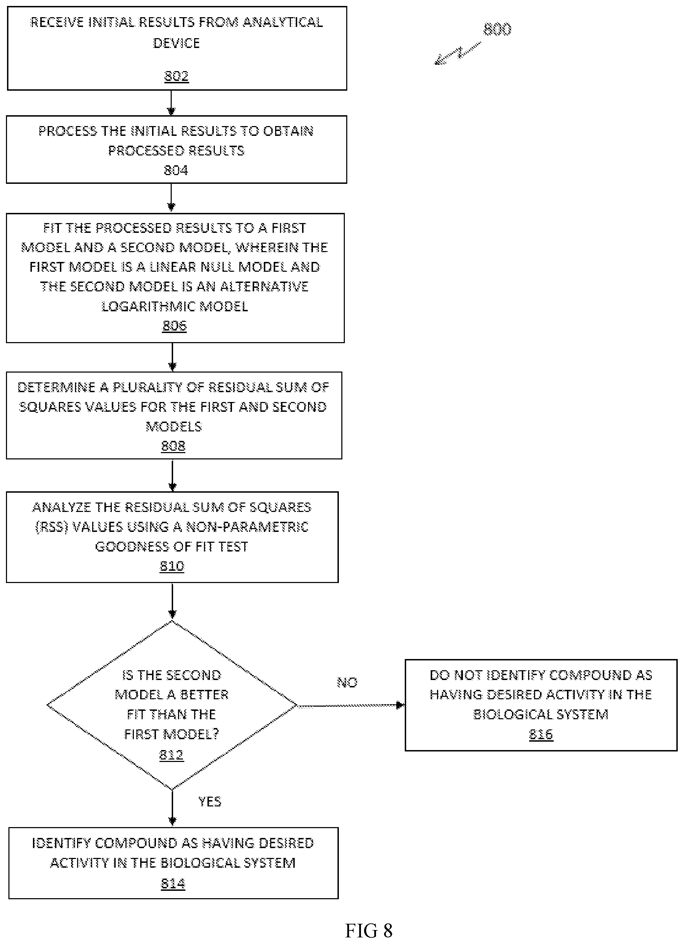

Aspects of the disclosure include a system for analyzing data from a biological assay comprising Real Time Cellular Thermal Shift Assay (RT-CETSA), differential scanning fluorimetry, thermal shift analysis, intrinsic fluorescence differential scanning fluorimetry, or nano differential scanning fluorimetry (nanoDSF).. Such aspects may include a memory storing instructions; and one or more processors that, responsive to executing the instructions are configured to carry out the aforementioned steps in the analytical methods described herein. A flow diagram illustrating a method **800** for carrying out such aspects for a single analyte is provided as Fig. 8.

At step **802,** the system receives initial results from the analytical device. The initial results may be read from local memory or received from a remote node via a network interface. The initial results may be in any appropriate form based on the nature of the biological assay. For example, in the context of RT-CETSA, the initial results may be in the form of an image file, such as a TIFF file or a JPEG file. In such aspects, the image file reflects the luminescence detected by the analytical device across the temperature gradient. Accordingly, the initial data may comprise multiple image files, each corresponding to a temperature step detected in the assay and depicting the detected luminescence of the various wells in, e.g., a 384 well plate.

At step **804,** the initial results are processed by the system as described above to obtain processed results. In this regard, initial results may be processed in any manner necessary to render the results in a suitable form for analysis via the disclosed methods. Such processing may include formatting, organizing, converting, transforming (e.g., subtracting background), and normalizing the results. For example, in aspects of the present disclosure wherein the biological assay is RT-CETSA, processing may involve organizing the images by assigning well addresses and by splitting the data into smaller sets, for example, multiple 96-well sets. Additional processing may be carried out by suitable software programs and scripts, as will be familiar to those skilled in the art. For example, initial results may be processed by MOLTENPROT.

At step **806,** for each tested concentration of the each of the one or more analytes, at each tested value of the first independent variable, the system fits the processed results to a first model and a second model, wherein the first model is a linear null model and the second model is an alternative logarithmic model. In aspects, the linear model is constrained to a slope of 0. In aspects, the alternative logarithmic model may be a log-logistical model with 3-5 parameters.

At step **808,** the system determines a plurality of residual sum of squares (RSS) values for the first and second models for each of the tested concentrations for the one or more analytes at each tested value of the first independent variable. In aspects, the residual sum of squares (RSS) values are calculated for both of the models for each experimental condition, e.g., for each value of the first independent variable and second independent variable. This process is then iterated for all tested values of the first independent variable. For example, in the context of an RT-CETSA assay, for any given temperature, both models are applied to the processed results (comprising collected luminescence data as described above) for each tested concentration of the analyte, in other words, the two models are applied to the dose response curve at the given temperature. The RSS value is then calculated at the given temperature. The process is then repeated at each temperature at which data was collected.

At step **810,** the system analyzes the RSS values for the single analyte using a non-parametric goodness of fit test. In aspects, the RSS values for both models are compared at each value for the first independent variable using a non-parametric goodness-of-fit test, for example a Mann-Whitney U non-parametric test.

At step **812,** the system analyzes the results of the goodness of fit test to determine whether the analyte has the desired activity by determining whether the second model is a better fit than the first model at one or more tested values for the first independent variable. According to aspects of the disclosure, the analyte is determined to have the desired activity in the biological system if the Mann-Whitney U test indicates that the second model is a better fit at a given value of the first independent variable than the first (null) model at one or more of the tested values of the first independent variable. For example, in the analysis of RT-CETSA data, an analyte will be identified as having the desired activity if, at one or more temperatures, the log logistic model is a better fit than the null model based on the Mann-Whitney U test.

At step **814,** responsive to determining for the single analyte that the second model is a better fit than the first model based on the analysis using the non-parametric goodness of fit test, identify the single analyte as having desired activity in the biological system. For example, an analyte may be identified as having the desired activity, if at one more value for the first independent variable, the concentration response data for the analyte better fits the log logistic model than the null model. For example, in the context of RT-CETSA an analyte may be identified as having the desired activity if the results for at least one temperature point fit the log logistic model better than the null model. In addition, following step **814,** the system and method can involve calculating EC₅₀ of the analyte at temperature corresponding to the maximal RSS value, which is not explicitly depicted in Fig. 8.

At step **816,** responsive to a determination that the second model is not a better fit based on the non-parametric goodness of fit test, the system identifies the analyte as not having the desired activity in the biological system. In aspects wherein the biological assay is RT-CETSA, an analyte may be identified as not having the desired activity where the null model is a better fit across the temperature gradient.

In aspects, the system, having executed the method illustrated in Fig.8 for a single analyte, may repeat the process for all analytes in a given set of initial data. The system may further calculate an EC₅₀ value for one or more of the analytes as disclosed above.

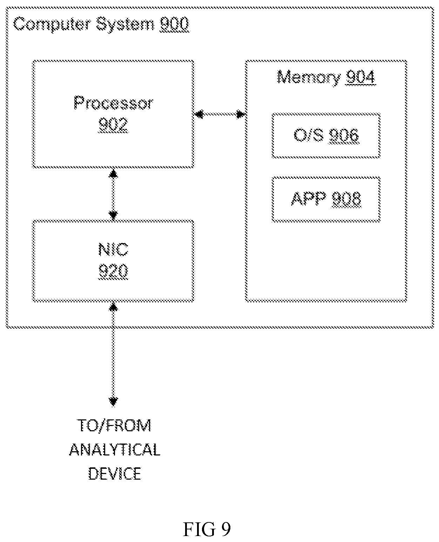

In aspects, the system for data analysis may comprise a computer system. An exemplary computer system **900** is illustrated in Fig. 9. The computer system **900** includes a processor **902,** a memory **904,** and a network interface controller (NIC) **920.** The memory **904** can include a volatile memory such as a dynamic random-access memory (DRAM) and a non-volatile memory such as a hard disk drive (HDD) or a solid-state drive (SSD). The processor 902 can execute instructions that cause the computer system **900** to implement the functionality or operations described with respect to any of the disclosed aspects.

Each of the components **902, 904,** and **920** can be interconnected, for example, using a system bus to enable communications between the components. The processor **902** is capable of processing instructions for execution within the system **900.** The processor **902** can be a single-threaded processor, a multi-threaded processor, a vector processor or parallel processor that implements a single-instruction, multiple data (SIMD) architecture, or the like. The processor **902** is capable of processing instructions stored in the volatile memory **904.** In some embodiments, the volatile memory **904** is a dynamic random-access memory (DRAM). The instructions can be loaded into the volatile memory **904** from a non-volatile storage, such as a Hard Disk Drive (HDD) or a solid-state drive (not explicitly shown) or received via the network. In an embodiment, the volatile memory **904** can include instructions for an operating system **906** as well as one or more applications **908.** It will be appreciated that the application(s) can be configured to provide the functionality of one or more components of the system. The NIC **920** enables the computer system **900** to communicate with other devices over a network, including a local area network (LAN) or a wide area network (WAN) such as the Internet. In aspects, the system communicates with an analytical device as indicated.

It will be appreciated that the computer system **900** is merely one exemplary computer architecture and that the processing devices implemented in the disclosed system can include various modifications such as additional components in lieu of or in addition to the components shown in Fig. 9. For example, in some embodiments, the computer system **900** can be implemented as a system-on-chip (SoC) that includes a primary integrated circuit die containing one or more CPU cores, one or more GPU cores, a memory management unit, analog domain logic and the like coupled to a volatile memory such as one or more SDRAM integrated circuit dies stacked on top of the primary integrated circuit dies and connected via wire bonds, micro ball arrays, and the like in a single package (e.g., chip). In another embodiment, the computer system **900** can be implemented as a server device, which can, in some embodiments, execute a hypervisor and one or more virtual machines that share the hardware resources of the server device.

It is noted that the techniques described herein may be embodied in executable instructions stored in a computer readable medium for use by or in connection with a processor-based instruction execution machine, system, apparatus, or device. It will be appreciated by those skilled in the art that, for some embodiments, various types of computer-readable media can be included for storing data. As used herein, a "computer-readable medium" includes one or more of any suitable media for storing the executable instructions of a computer program such that the instruction execution machine, system, apparatus, or device may read (or fetch) the instructions from the computer-readable medium and execute the instructions for carrying out the described embodiments. Suitable storage formats include one or more of an electronic, magnetic, optical, and electromagnetic formats. A non-exhaustive list of conventional exemplary computer-readable medium includes: a portable computer diskette; a random-access memory (RAM); a read-only memory (ROM); an erasable programmable read only memory (EPROM); a flash memory device; and optical storage devices, including a portable compact disc (CD), a portable digital video disc (DVD), and the like.

It should be understood that the arrangement of components illustrated in the attached Figures are for illustrative purposes and that other arrangements are possible. For example, one or more of the elements described herein may be realized, in whole or in part, as an electronic hardware component. Other elements may be implemented in software, hardware, or a combination of software and hardware. Moreover, some or all of these other elements may be combined, some may be omitted altogether, and additional components may be added while still achieving the functionality described herein. Thus, the subject matter described herein may be embodied in many different variations.

To facilitate an understanding of the subject matter described herein, many aspects are described in terms of sequences of actions. It will be recognized by those skilled in the art that the various actions may be performed by specialized circuits or circuitry, by program instructions being executed by one or more processors, or by a combination of both. The description herein of any sequence of actions is not intended to imply that the specific order described for performing that sequence must be followed. All methods described herein may be performed in any suitable order unless otherwise indicated herein or otherwise clearly contradicted by context.

### EXAMPLES

The following examples should not be construed as in any way limiting the scope of the present disclosure.

### EXAMPLE 1

This example describes the development of a thermally stable nanoLuciferase-based reporter molecule for use in Real Time CETSA experiments.

The nanoLuciferase (NanoLuc) enzyme is a commonly used and highly-luminescent 19.1 kDa reporter molecule. However, its lower melting temperature (58 °C) would mask most ligand-induced stabilization and falsely shift the apparent temperature of aggregation, or T_{agg}, because the melting of NanoLuc would drive aggregation rather than the melting of the protein of interest.

To develop a thermally stable NanoLuc based reporter molecule, the inventors investigated the characteristics of fragments of NanoLuc. 11S refers to the LgBit fragment of NanoLuc, the amino acid sequence of which is provided as SEQ ID NO: 18.

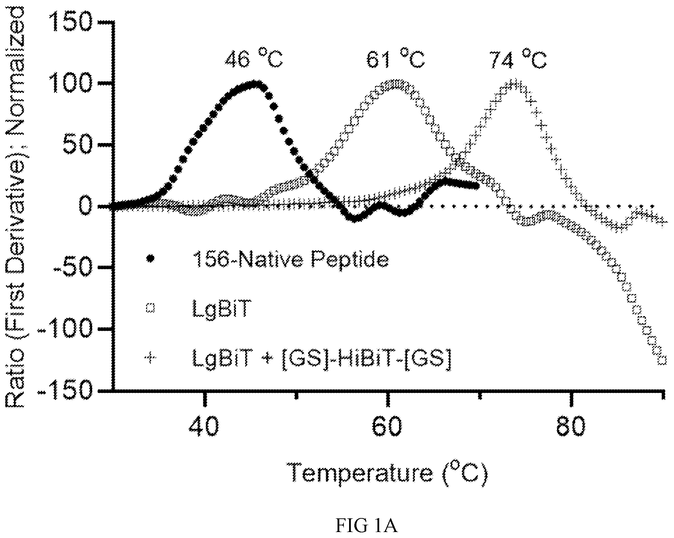

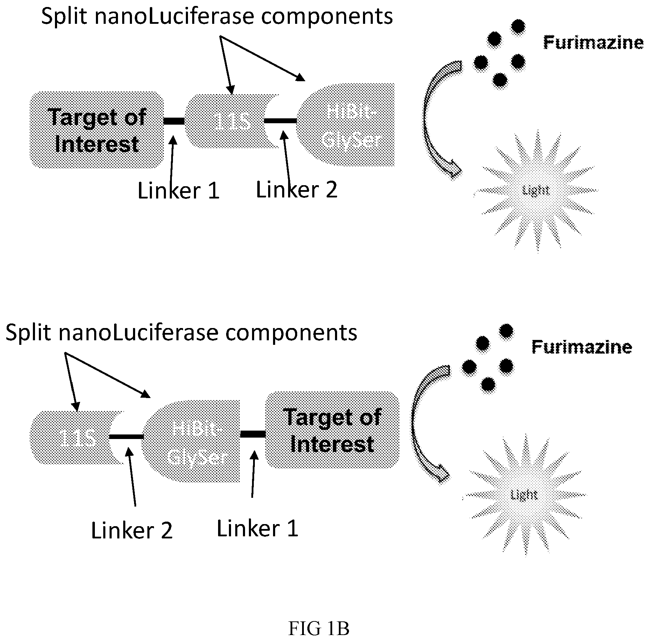

The LgBiT (11s) fragment of NanoLuc was attached to a peptide comprising the HiBit fragment of NanoLuc with a GlySer linker. This resulted in an increased melting point relative to the LgBiT fragment by itself and 156+Native peptide. (Fig. 1A.) A graphical depiction of such a reporter region is provided as Fig. 1B.

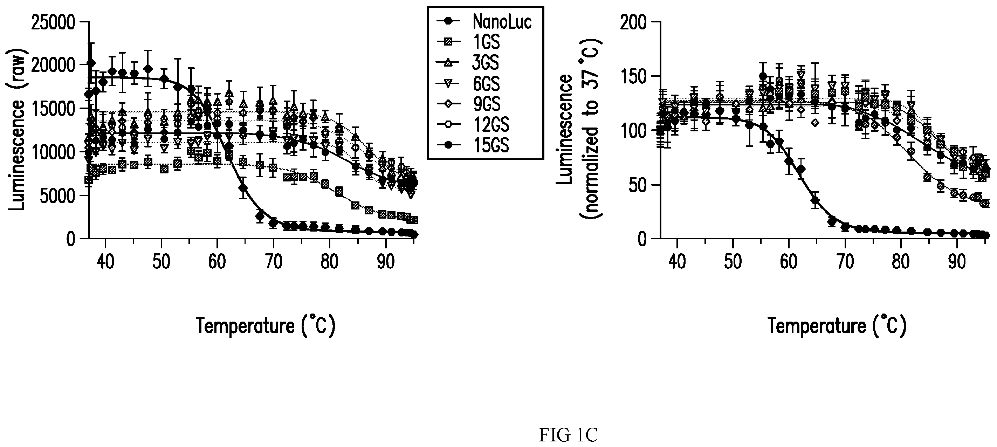

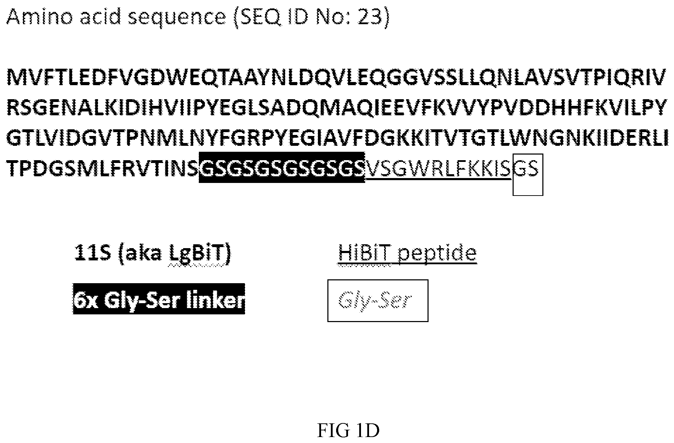

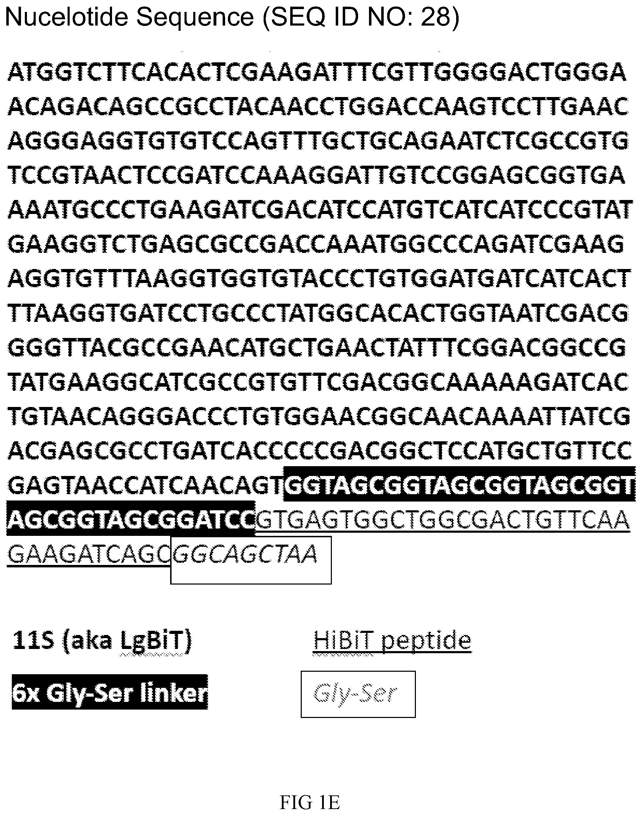

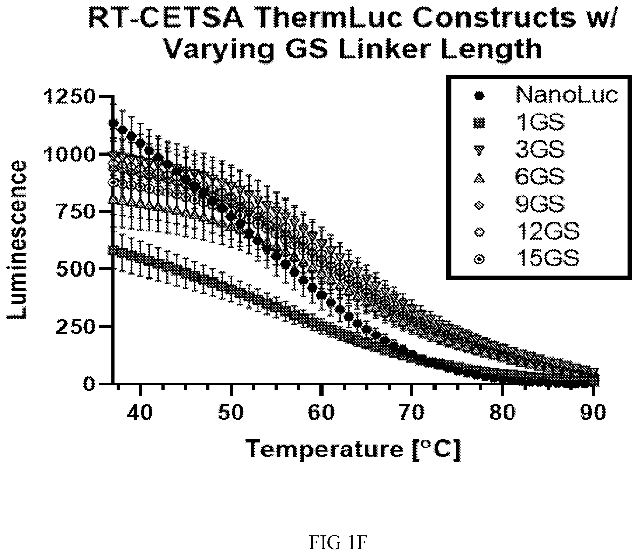

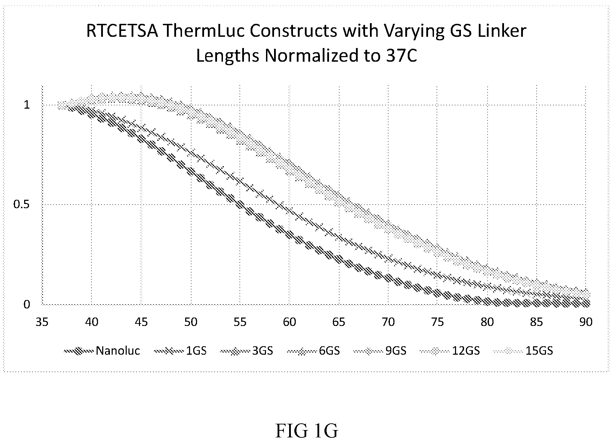

To develop the reporter molecule, the melting profile of 11s-86b fusion proteins with varying Gly-Ser linker lengths were obtained in cells and compared with NanoLuc. (Figs. 1C, 1F and 1G.) When the size of the linker was increased to 3 GlySer repeats or higher between the two fragments, the resultant fusion protein displayed minimal melting over the temperature ramp range commonly used in CETSA experiments. The reporter molecule comprising the LgBiT and HiBiT-GlySer joined by a 6X GlySer linker is referred to as "ThermLuc" and the full sequences are provided as SEQ ID NO: 23 (peptide), and a nucleotide sequence encoding ThermLuc is provided as SEQ ID NO: 28. (See Figs 1D and 1E, respectively.)

Importantly, although ThermLuc displays a marked decrease in the luminescent signal compared to native NanoLuc (see Fig. 1C), the signal is still strong enough to enable quantification with commonly used lab Charge-coupled Devices (CCDs).

### EXAMPLE 2

This example describes the development of a device suitable for conducting RT-CETSA experiments.

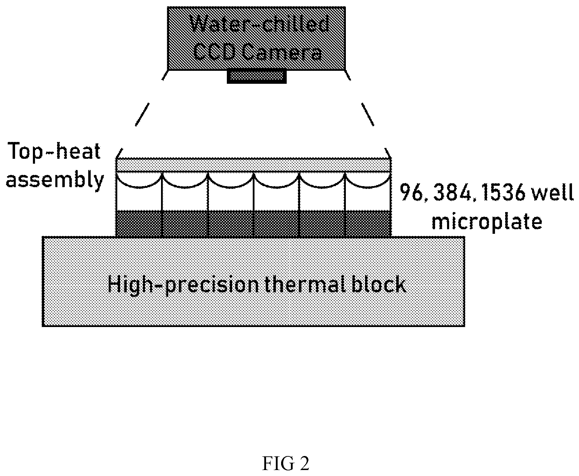

As no RT-PCR machine on the market is designed for luminescence capture, existing devices are not suitable for carrying out RT-CETSA experiments. RT-CETSA requires a high-precision and high-speed PCR thermal block capable of handling several plate formats (e.g. 96 well, 384 well, etc.), and a sensitive CCD or CMOS camera able to capture luminescence. The configuration is depicted visually in Fig. 2.

An RT-CETSA prototype was built out of a commercially-available high-throughput RT-PCR machine, the Roche LC480 (Product No. 05015278001). Excitation and emission filters were removed to maximize signal, and the camera was replaced with a water-cooled Hamamatsu Orca II CCD (C11090-22B) capable of sensitive luminescence capture. Additionally, Software tools were created to capture luminescence data from 384 well plates and perform analysis workflow to visualize real-time CETSA datasets.

### EXAMPLE 3

This example describes the RT-CETSA assay and presents results obtained thereby.

In an RT-CETSA experiment, cells are transfected with a plasmid vector encoding a target protein of interest coupled with the ThermLuc reporter molecule. The target protein of interest is coupled to ThermLuc with a first linker peptide sequence GSGGGGS (SEQ ID NO: 1). The target-ThermLuc construct is then expressed in the cells.

The transfected cells are loaded onto a plate (e.g., a 96 well plate or a 384 well plate). Furimazine is then added to the samples, and the plate is exposed to a heat ramp via the high precision, high speed PCR thermal block. The luminescent signal of the intact target protein construct is captured in real time by the CCD camera. When a given temperature is reached, the target protein of interest will unfold and aggregate, and the luminescence will fade. The temperature at which 50% of the protein has aggregated is referred to as T_{agg}. Accordingly, the assay allows for full aggregation profiles of multiple proteins to be captured in parallel. Further, as heat induced aggregation can be altered by a small molecule binding to the target protein, ligand induced thermal shifts can also be observed.

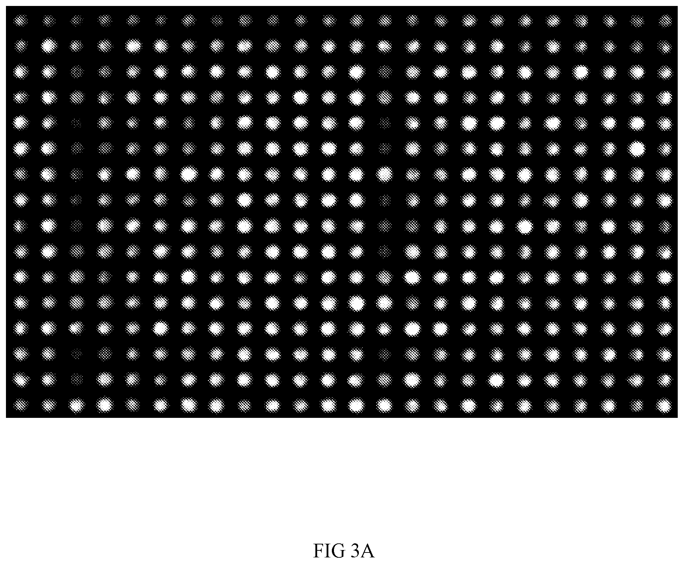

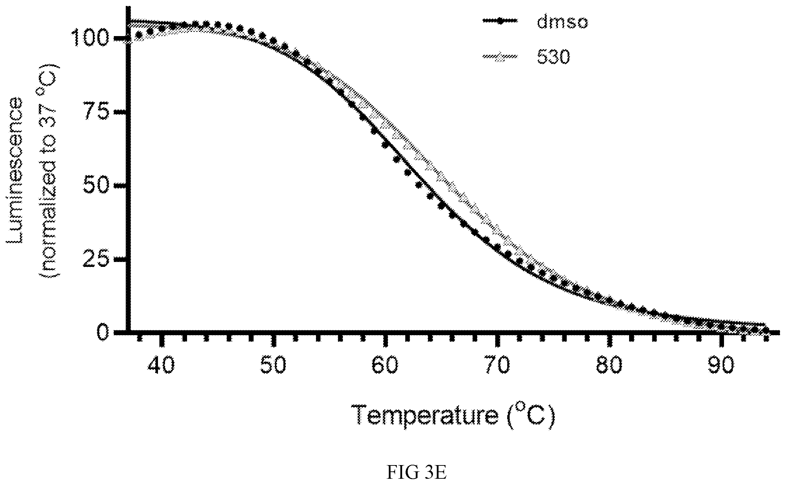

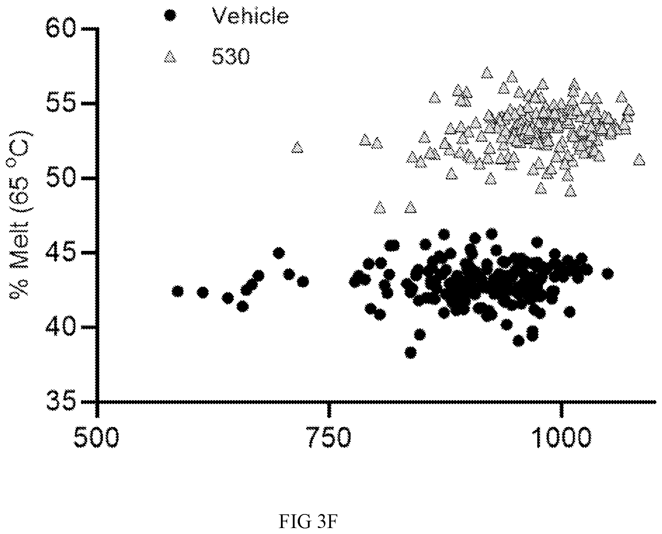

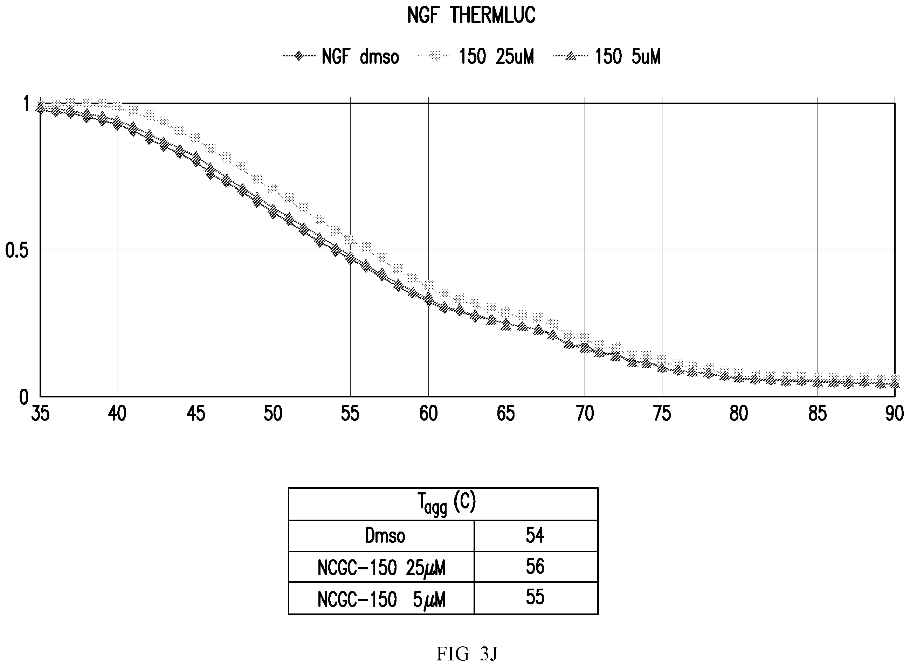

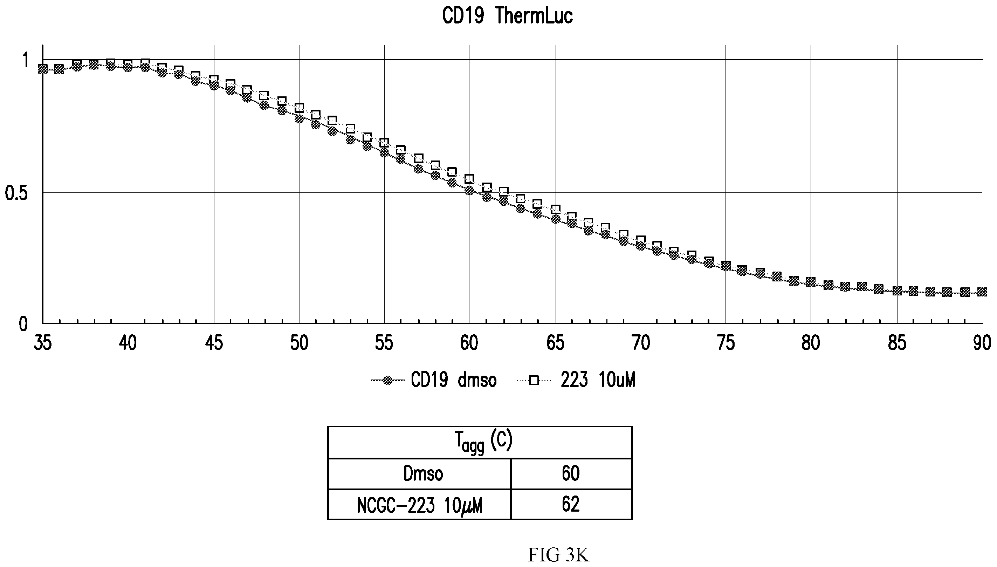

In an example of such an assay, HEK293T cells in a plate were transfected with LDHA-ThermLuc plasmid and then treated with known LDHA binders and non-binders. A still image depicting the plate containing the LDHA- ThermLuc transfected HEK293T cells during the assay is provided as Fig. 3A. This still image is representative of a single time point in the continuous, real-time, visualization of luminescence provided by RT-CETSA. Over the course of a RT-CETSA experiment, the observed luminescence for each well gradually decreases to background luminescence values.

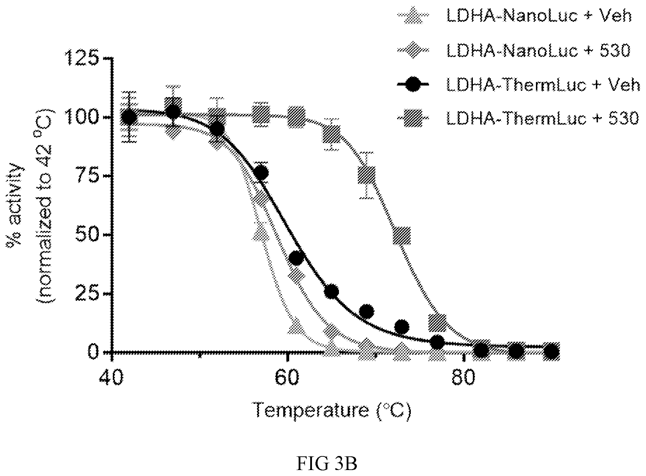

Results of an experiment utilizing HEK293T cells wherein the target protein of interest is LDHA is provided as Fig. 3B. In this assay, LDHA was fused with either nanoLuciferase or ThermLuc protein and was transfected into HEK293T cells. The cells were then exposed to different temperatures. The stabilization of LDHA (T_{agg} ~ 60 °C) with a known LDHA inhibitor is masked when using NanoLuc as a reporter, because NanoLuc is driving the aggregation of the fusion complex. Specifically, the shift in melt temperature after treatment with known binder 530 is masked (ΔTₘ=2.0°C) by the lower T_{agg} of nanoLuciferase, but it becomes apparent with the ThermLuc protein (ΔTₘ=12.5°C). Accordingly, this assay shows that the ThermLuc protein was not the driver for aggregation of the target of interest.

RTCETSA can produce dose-response curves for small molecules against the target(s) of interest. T_{agg} values from the LDHA RT-CETSA experiment are derived from the luminescent signal and plotted against compound concentration. Multiple dose-responses are detected, in good agreement with prior art on these compounds against LDHA.

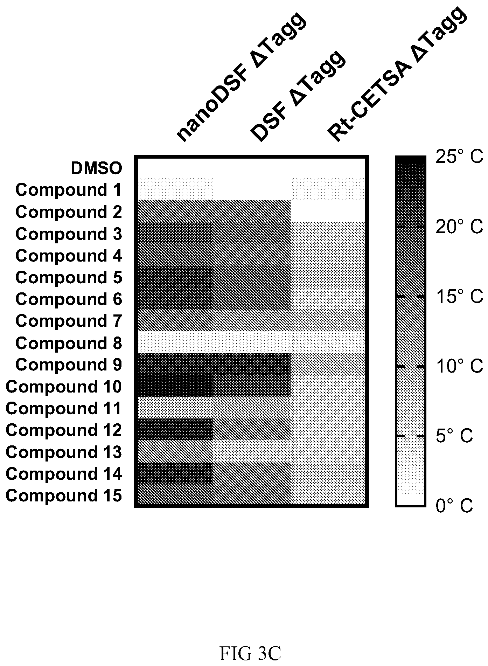

The RT-CETSA method detected binders with good correlation with other biophysical methods. The ΔT_{agg} values for the LDHA experiment produced using RT-CETSA are similar to the Tm values using differential scanning fluorimetry methods, which quantify protein melting either by detecting intrinsic amino acid fluorescence (nanoDSF) or a reporter dye (DSF). Some compounds may show binding in the DSF assay with purified protein, but not CETSA, because they do not bind the target in cells, for instance because they lack membrane permeability. These results are depicted in Figure 3C.

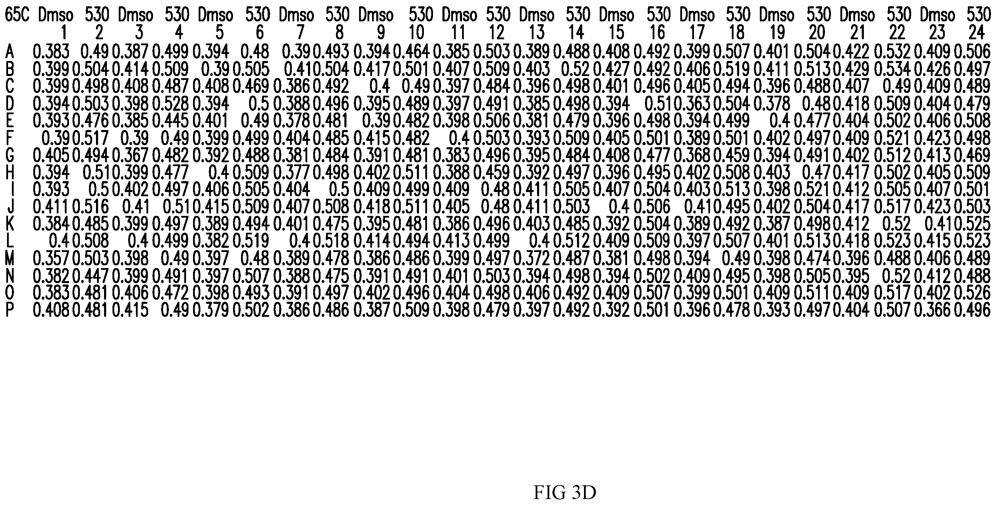

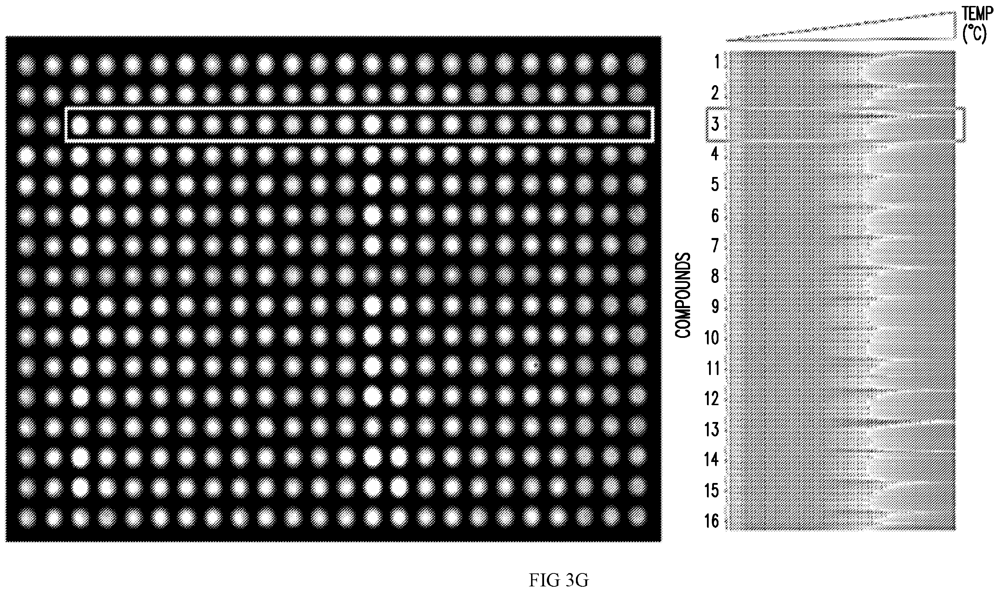

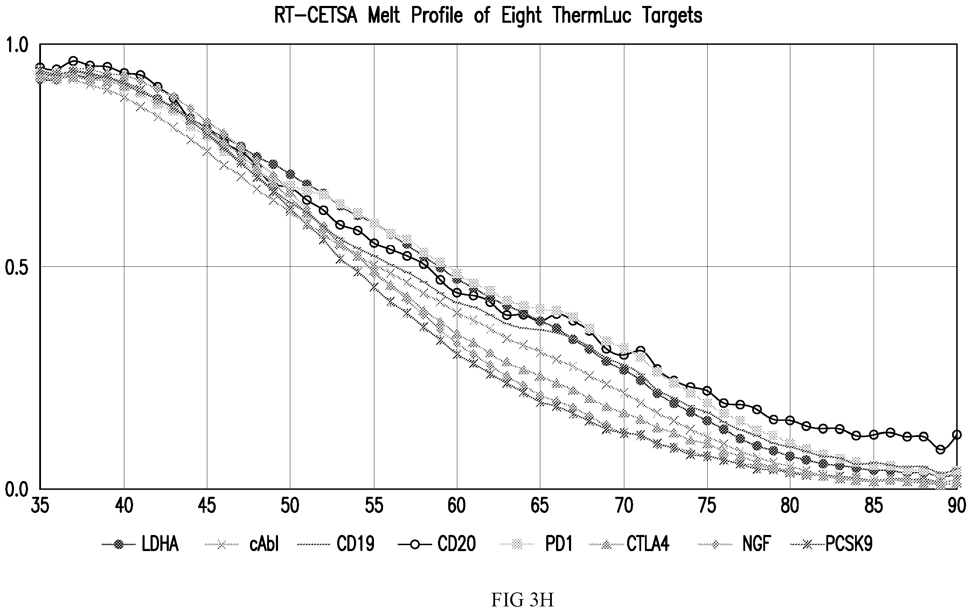

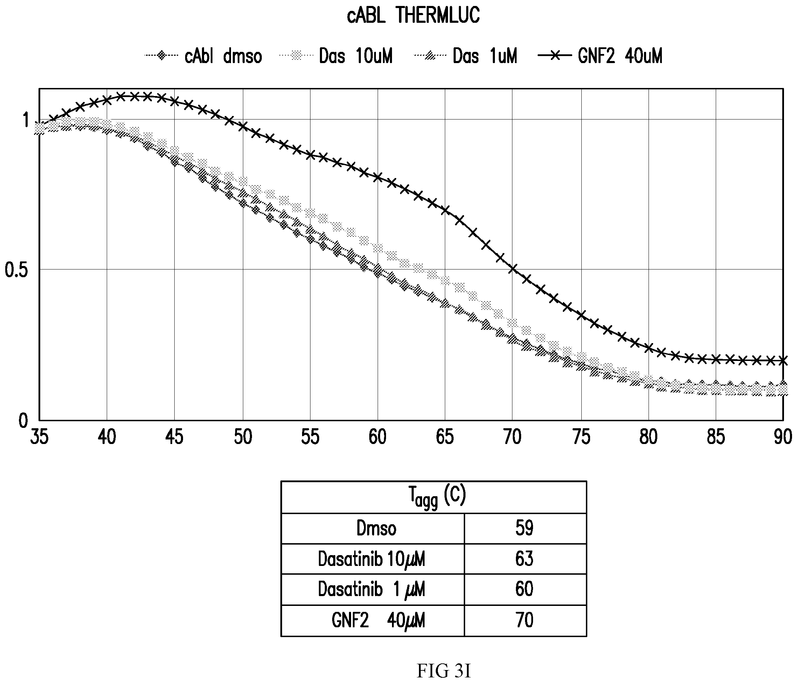

Depictions of additional data obtained demonstrating the RT-CETSA assay are provided as Figs. 3D-3K, and are described briefly, herein. Figures 3D-3F depict thermal shifts across a plate. Fig. 3G presents a visual depiction of data indicating that LDHA inhibitors show a dose dependent shift. Fig. 3H depicts the RT-CETSA melt profile of eight ThermLuc target proteins of interest. Finally, Figs. 3I-3K depict target engagement in RT-CETSA using additional protein targets of interest.

### EXAMPLE 4

This example provides comparisons between RT-CETSA and traditional CETSA.

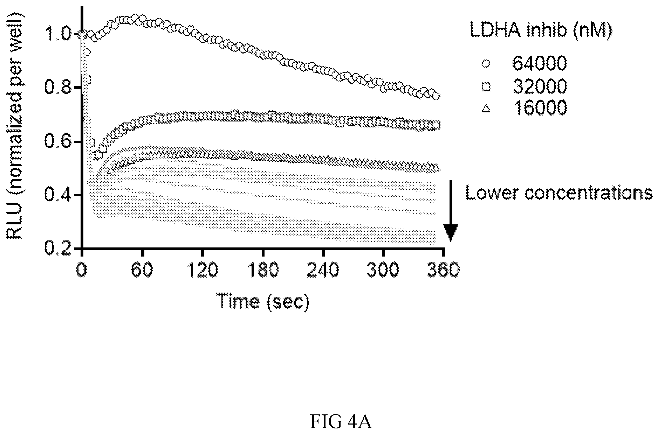

The original CETSA technique calls for application of heat for 3.5 minutes to samples. However, using the RT-CETSA method, it was demonstrated that melting of the target takes place within seconds of the application of heat. (Fig. 4A). Accordingly, RT-CETSA allows for a real-time read out of target melting that cannot be obtained with other CETSA techniques.

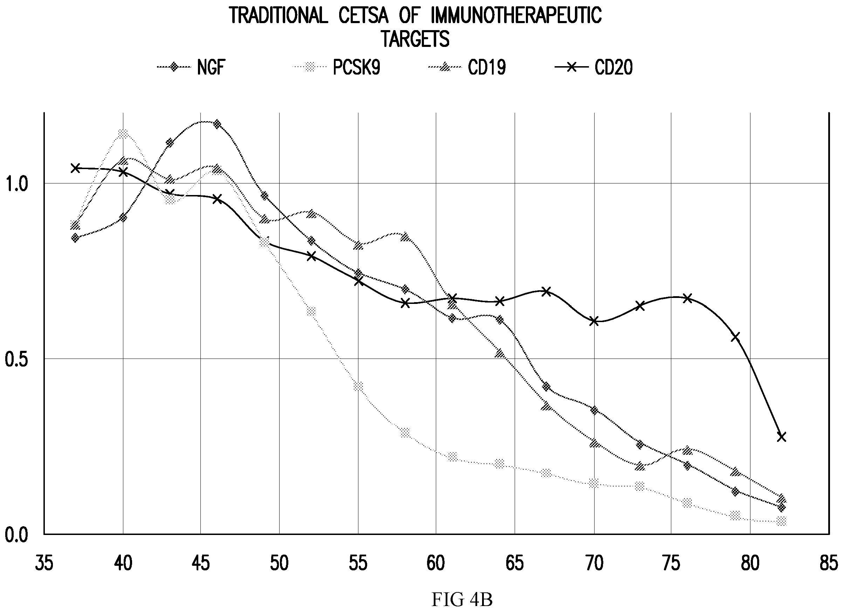

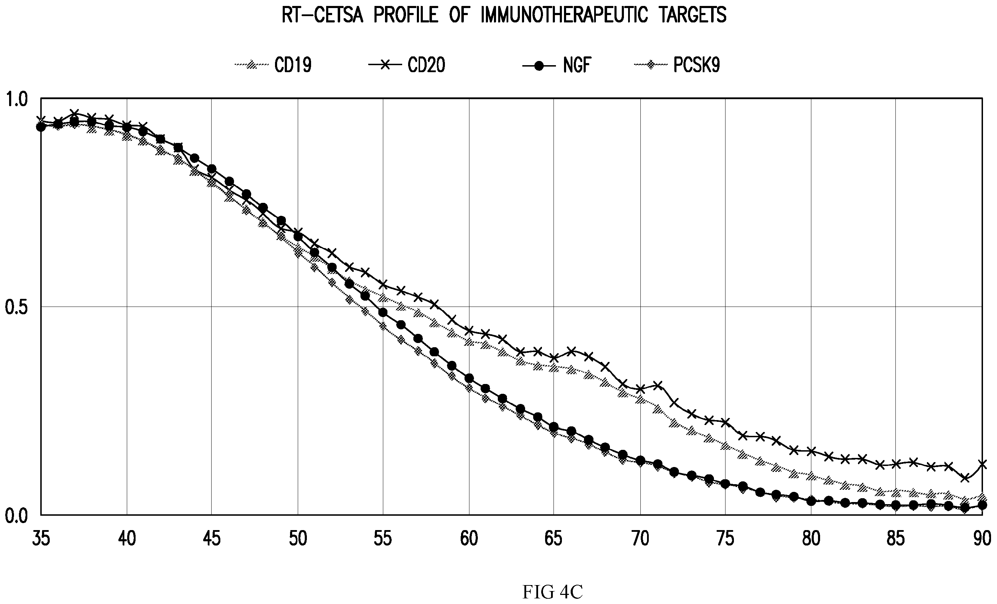

An additional comparison is depicted in Figs. 4B-C. Briefly, Figs 4B and 4C demonstrate the profile of immunotherapeutic targets obtained with traditional CETSA as compared to RT-CETSA.

### EXAMPLE 5

This example demonstrates the utility of RT-CETSA in profiling multiple target proteins.

All previous CETSA methods require optimization for each protein target, but RT-CETSA allows for multiple targets with variable melting profiles to be screened in the same experiment with less initial optimization.

RT-CETSA will allow multiple proteins (e.g., multiple members of a target class) to be profiled in the same experiment without extensive optimization. Multiple targets can be monitored in parallel even if they have different aggregation profiles. For example, data obtained from a multitarget RT-CETSA experiment will allow entire families of proteins to be profiled against a panel of compounds, i.e. a family of kinases or methyl transferases against a known activator or inhibitor for off-target or intra-family engagement.

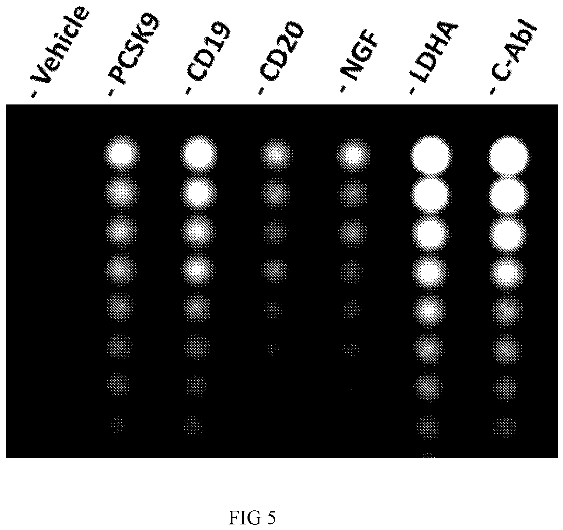

An example of a multi-target readout obtained by RT-CETSA is provided as Fig. 5.

### EXAMPLE 6

This example describes the utility of a universal acceptor plasmid to improve the convenience and ease of use of RT-CETSA.

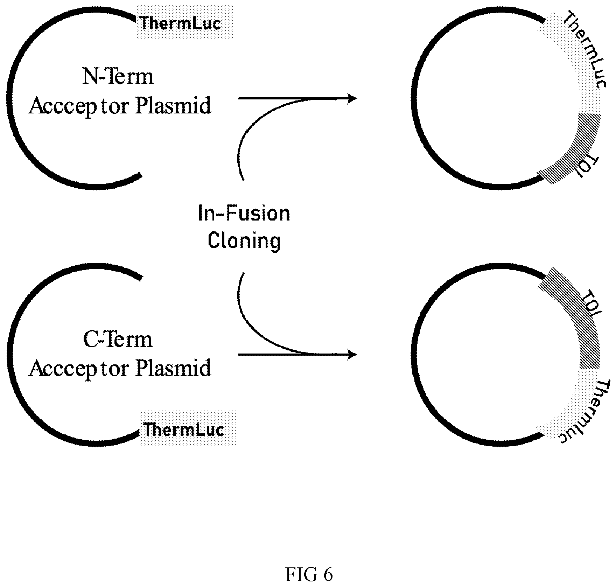

ThermLuc is inserted into a pcDNA3.1 vector with proper In-Fusion (Takara Bio) homologous sequences, containing a BamHI restriction site (encoding Gly-Ser) at the junction between ThermLuc and the target. (Fig. 6.) This will allow for researchers to easily clone in their target of interest without any unwanted, extra base pairs, to construct N-terminal or C-terminal fusion proteins. The universal acceptor plasmids expedite the process of cloning for RT-CETSA.

### EXAMPLE 7

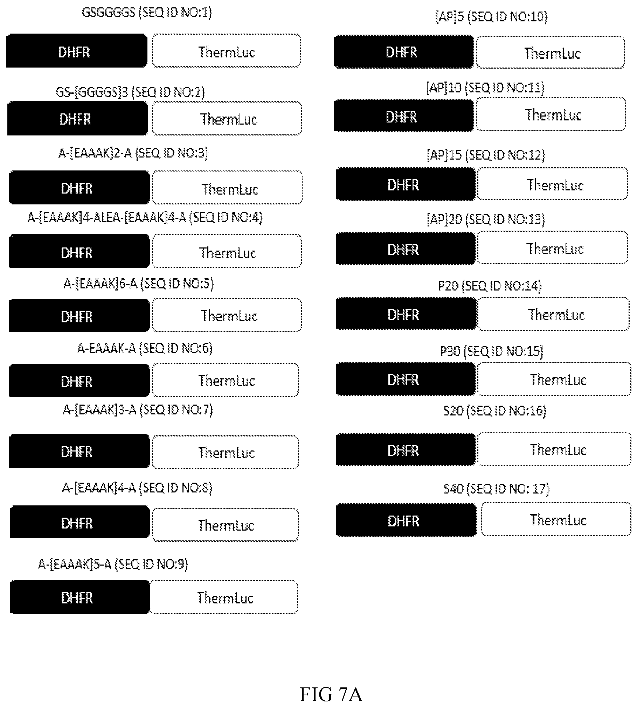

This example describes dihydrofolate reductase (DHFR)-ThermLuc reporter constructs. Various constructs were prepared with various first peptide linker region sequences between the target of interest (i.e., DHFR) and the reporter molecule as summarized in Figure 7A.

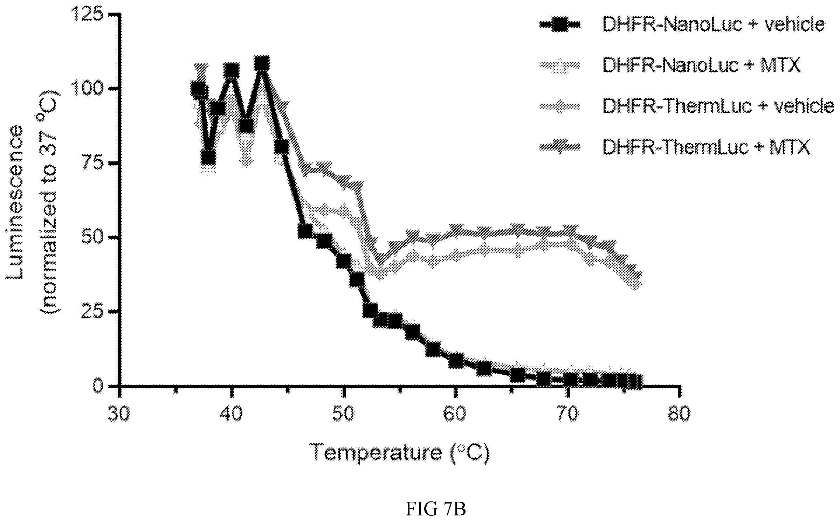

Cellular thermal melt profiles of DHFR fusion proteins were determined. An elevated melting temperature was observed for NanoLuc and ThermLuc constructs relative to previously reported values for unlabeled or SplitLuc DHFR. However, as illustrated in figure 7B, only partial melting was observed in the case of the ThermLuc constructs (as indicated by the fact that higher luminescence was observed at higher temperatures relative to NanoLuc constructs). This reveals intramolecular thermal stabilization conferred by ThermLuc.

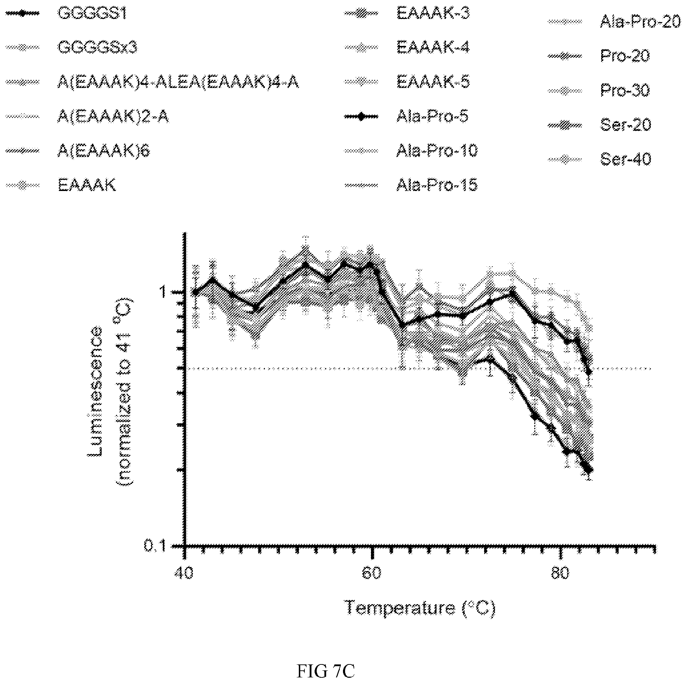

Fig 7C is a graph depicting the thermal stability of DHFR-ThermLuc fusions when separated by various linkers/spacers (i.e., the various spacers summarized in Figure 7A). This reveals the melting temperature of fusion proteins that show altered behavior as ThermLuc fusions with, e.g., a short GlySerGlyGlyGlyGlySer first peptide linker (SEQ ID NO: 1), can be further altered by varying first peptide linker.

### EXAMPLE 8

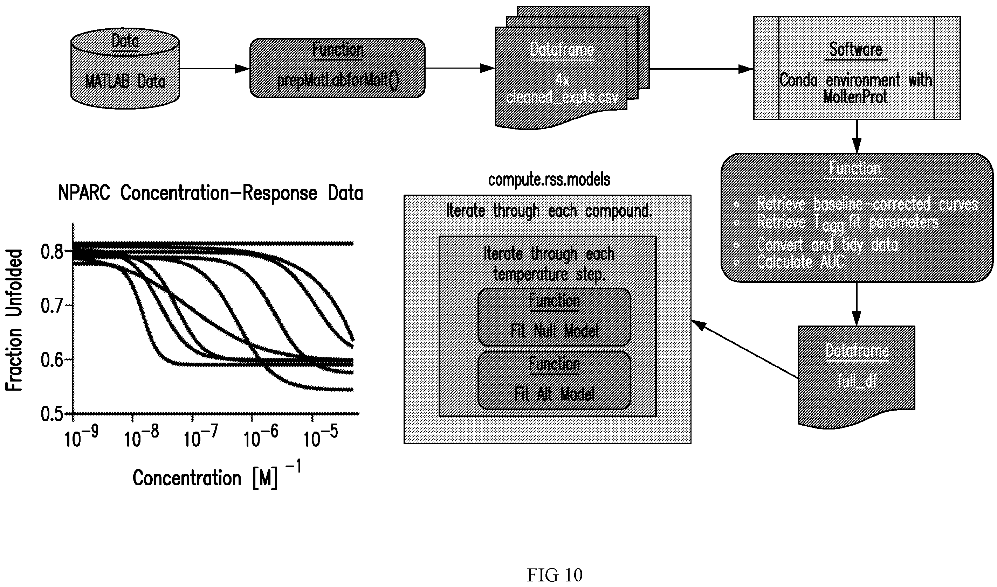

This example details an exemplary workflow for applying the disclosed analysis methods to data generated by RT-CETSA. An illustration of the exemplary workflow is provided as Figure 10.

In this workflow, data generated during a RT-CETSA experiment initially consists of TIFF images equal to the number of temperature steps detected. This data is then processed as described below.

The images are parsed using a MATLAB script that sums the total luminescence values for each well across each image, providing data in single .csv file for processing. The RT-CETSA script prepares the RT-CETSA data for further analysis according to the disclosed methods using MOLTENPROT (CSSB, Hamburg, Germany) by assigning well addresses, temperatures for each reading, and splitting the 384 wells of data (corresponding to the 384 well plates used in the RT-CETSA assay) into 4 96-well files (MOLTENPROT is limited to processing 96 wells of data at a time). Using MOLTENPROT baseline-estimation and curve fitting are performed using supplied parameters (chosen after optimization using controls). The RT-CETSA script retrieves and organizes the baseline-corrected curve fits and Tagg values and creates a dataframe (df) to hold the values for each well at each temperature and assign sample IDs according to a supplied platemap. The script then calculates AUC using the supplied curves before assembling the full dataframe (full_df). Finally, parameter heatmaps, data frames, and individual concentration-response graphs are exported for end user analysis.

The compute.rss.models function then iterates each compound at each temperature step, fitting linear and 4-parameter log-logistic curves for the fraction unfolded data that represent the null and alternate models, respectively. The residual sum of squares for each model are derived for each temperature, and a Mann Whitney U test of the residuals is used to determine compounds that produce a significant shift in the melting curve from the DMSO control (or no Effect). P values are adjusted for significance to p = 0.05 / # of compounds tested.

### EXAMPLE 9

This example provides exemplary data and analysis from an RT-CETSA assay.

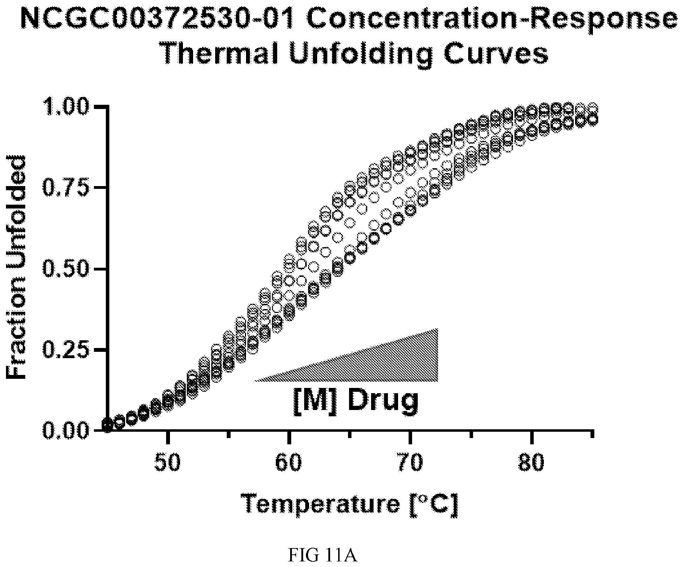

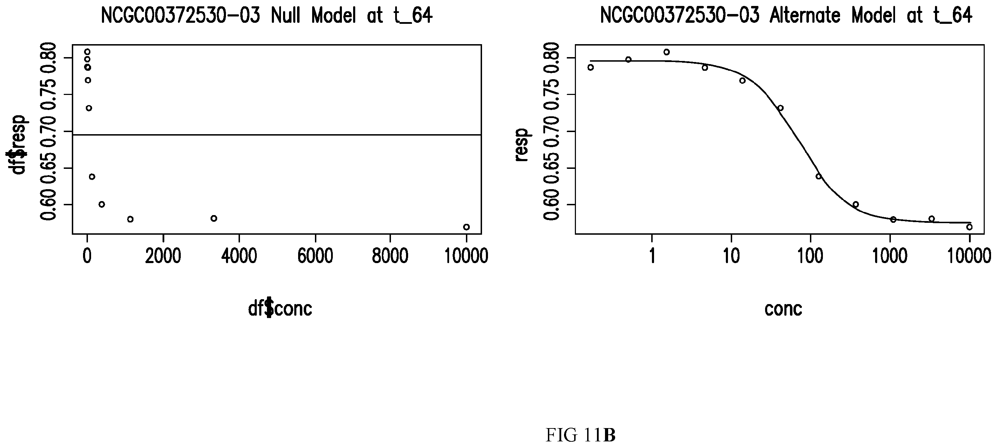

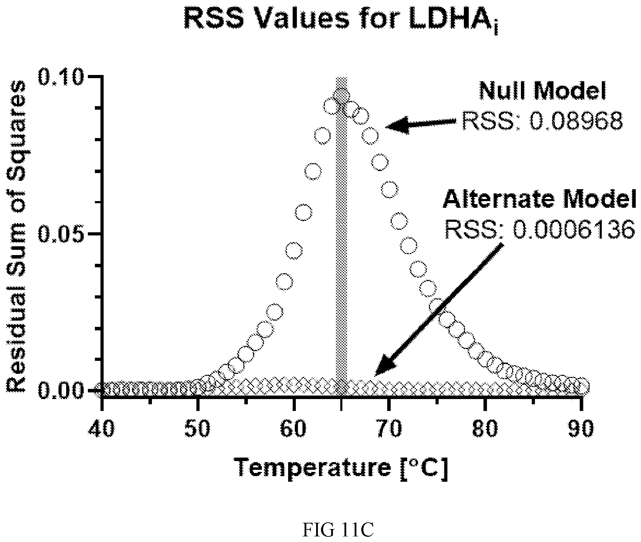

In this example, thermal unfolding data is presented from a single RT-CETSA experiment analyzing LDHA against a panel of 29 previously characterized LDHA inhibitors (i.e., "LDHAᵢ", see Rai et al., J Med Chem. 60(22): 9184-9204, 2017.) that has been processed and baseline-corrected in MOLTENPROT. (See Fig. 11A.) Both null (linear regression with slope = 0) and an alternate models (log-logistic 4 parameter fit) derived by the disclosed methods (i.e., the NPARC script) at 64 °C for the LDHAᵢ NCGC00372530-01 are presented as Fig. 11B. For the analysis, the fraction unfolded vs LDHAᵢ NCGC00372530-01 concentration values are fit using the null model and the alternate model for each temperature. The residual sum of squares (RSS) of the null and alternate models are calculated at each temperature. Figure 11C depicts the RSS values plotted against the temperature. As can be seen, the point of greatest difference occurs at 64 °C.

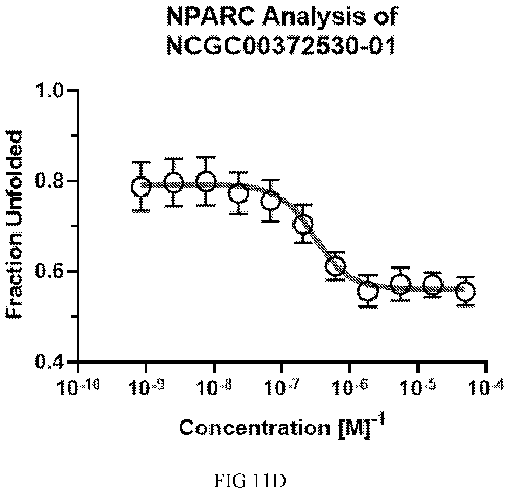

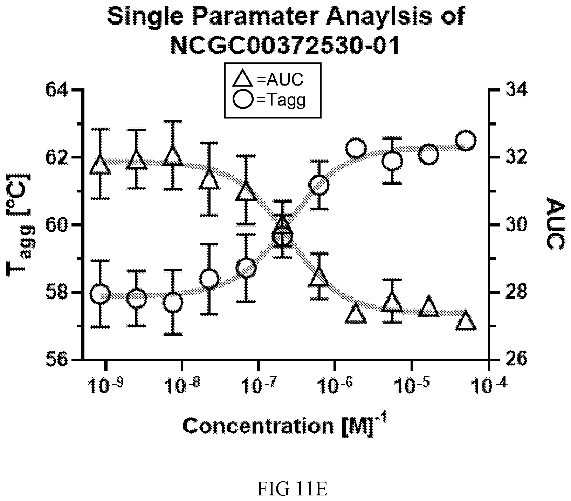

This point of maximal difference for NCGC00372530-01 at 64 °C is then used to determine the EC₅₀ for the compound with significant Mann-Whitney U statistical test. (See Fig. 11D.) The EC₅₀ of NCGC00372530-01 as determined by the disclosed method is 267.7 nM (95% CI 154.6 - 441.3 nM). EC₅₀ is derived by performing a log-logistic 4-parameter dose-response fit from the fraction unfolded values at the point of maximal RSS difference. The data shown is representative of three biological replicates (mean ± standard deviation). Fig. 11E depicts single parameter T_{agg} [249.6 nM (95% CI 136.3 - 438.0 nM)] and AUC [246.8 nM (95% CI 135.6 - 429.6 nM)] EC₅₀ values for NCGC00372530-01 concentration-response data. The data shown is representative of three biological replicates (mean ± standard deviation).

### EXAMPLE 10

This example provides additional data and analysis from an RT-CETSA assay.

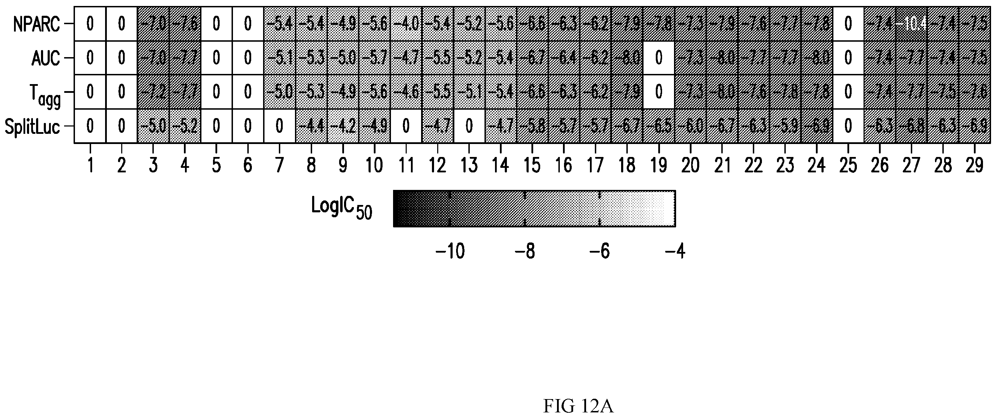

In Fig 12A, EC50 values for a partial multi-well plate of target engagement of LDHA inhibitors (n=3 replicates for each method) are presented. The first three rows depict data obtained from RT-CETSA assays. The top row presents data obtained using the disclosed methods (indicated by the label "NPARC"). The second and third rows depict RT-CETSA results obtained from known methods of analysis (i.e., T_{agg} and AUC.) The bottom row presents data obtained from an orthogonal target engagement assay, SplitLuc assay CETSA. Compounds with no detected binding are annotated as "0". As can be seen in column 19, the disclosed methods of analysis correctly identified a LDHA inhibitor that would have been missed by the single parameter T_{agg} and AUC approaches.

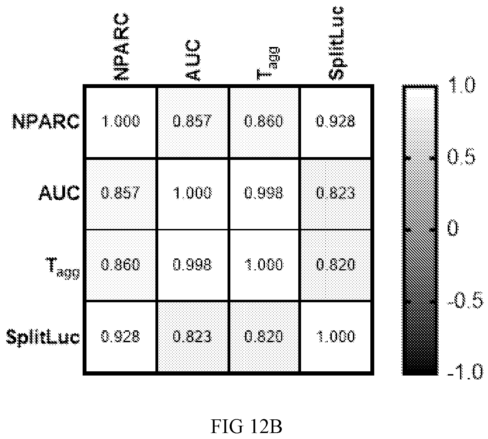

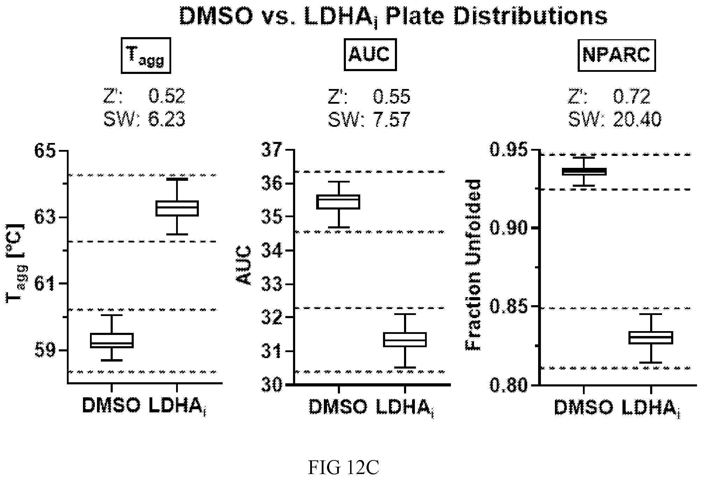

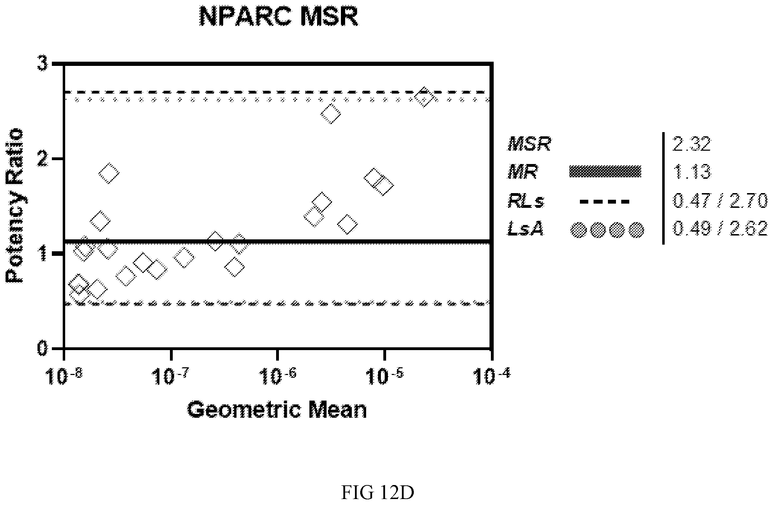

Fig. 12B presents spearman coefficients showing high correlation with single parameter analysis of RT-CETSA data and SplitLuc CETSA methods. All correlations were statistically significant (p<0.005, two-tail). In Fig. 12C, boxplot distributions of positive and negative controls using LDHA-ThermLuc are determined. These values are used to determine the Z' statistic and signal window using Tagg, AUC, and NPARC methods of analysis. Solid lines represent the means of each group, and dashed lines represent the ±3*SD for each control group. In Fig. 12D, testing of the minimum significant ratio (MSR) and related parameters further characterize the high reproducibility of potency estimates from the RT-CETSA method. The mean ratio (MR) is shown as a solid blue line, Limits of Agreement (LsA) in dashed red lines, and ratio limits (RL) in dashed green lines.

The use of the terms "a" and "an" and "the" and "at least one" and similar referents in the present disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. All methods described herein can be performed in any suitable order unless otherwise indicated herein or otherwise clearly contradicted by context.

## Claims

1. A system for analyzing data from a biological assay, wherein the biological assay comprises a biological system, and wherein the system for analyzing data identifies from one or more analytes those analytes demonstrating a desired activity in the biological system, wherein data from the biological assay is obtained using an analytical device, and wherein the data from the biological assay comprises results obtained at a plurality of experimental conditions, the experimental conditions varying with respect to tested values for a first independent variable and tested values for a second independent variable, wherein the tested values for the first independent variable collectively comprise a temperature gradient and wherein the second independent variable is the concentration of each of the one or more analytes present in each of the plurality of experimental conditions, and wherein the biological assay comprises Real Time Cellular Thermal Shift Assay (RT-CETSA), differential scanning fluorimetry, thermal shift analysis, intrinsic fluorescence differential scanning fluorimetry, or nano differential scanning fluorimetry (nanoDSF), the system comprising:
a memory storing instructions; and
one or more processors that, responsive to executing the instructions, are configured to:
**a)** receive initial results from the analytical device;
**b)** process the initial results to obtain processed results;
**c)** fit the processed results for each concentration of a single analyte to a first model and a second model at each tested value of the first independent variable, wherein the first model is a linear null model with a slope of 0, and the second model is an alternative logarithmic model;
**d)** determine a plurality of residual sum of squares (RSS) values for the first and second models at each tested value of the first independent variable;
**e)** analyze the plurality of RSS values using a non-parametric goodness of fit test at each tested value of the first independent variable;
**f)** responsive to determining for the single analyte that the second model is a better fit than the first model for at least one tested value of the first independent variable, identify the single analyte as having the desired activity in the biological system; and
**g)** repeat steps (c)-(f) for each of the one or more analytes.

2. A method for analyzing data from a biological assay, wherein the biological assay comprises a biological system, and wherein the system for analyzing data identifies from one or more analytes those analytes demonstrating a desired activity in the biological system, wherein data from the biological assay is obtained using an analytical device, and wherein the data from the biological assay comprises results obtained at a plurality of experimental conditions, the experimental conditions varying with respect to tested values for a first independent variable and tested values for a second independent variable, wherein the tested values for the first independent variable collectively comprise a temperature gradient and wherein the second independent variable is the concentration of each of the one or more analytes present in each of the plurality of experimental conditions, and wherein the biological assay comprises RT-CETSA, differential scanning fluorimetry, thermal shift analysis, intrinsic fluorescence differential scanning fluorimetry, or nanoDSF, the method comprising:
**a)** receiving initial results from the analytical device;
**b)** processing the initial results to obtain processed results;
**c)** fit the processed results for each concentration of a single analyte to a first model and a second model at each tested value of the first independent variable, wherein the first model is a linear null model with a slope of 0, and the second model is an alternative logarithmic model;
**d)** determining a plurality of RSS values for the first and second models at each tested value of the first independent variable;
**e)** analyzing the plurality of RSS values using a non-parametric goodness of fit test at each tested value of the first independent variable;
**f)** responsive to determining for the single analyte that the second model is a better fit than the first model for at least one tested value of the first independent variable, identifying the single analyte as having the desired activity in the biological system; and
**g)** repeating steps (c)-(f) for each of the one or more analytes.

3. The system of claim 1 or the method of claim 2, wherein the non-parametric goodness of fit test is a Mann-Whitney U test.

4. The system or method of any one of claims **1-3,** wherein the alternative logarithmic model is a log-logistic fit model with 3-5 parameters.

5. The system or method of claim **4,** wherein the alternative logarithmic model is a log-logistic fit model with 4 parameters.

6. The system or method of any one of claims **1-5,** wherein the one or more processors are further configured to calculate an EC₅₀ value for the single analyte by plotting the RSS values from the first and second models at each value of the first independent variable, and then fitting the second model to the processed results at the value of the first independent variable where the amount of RSS difference between the first and second models is greatest.

7. The system or method of any one of claims **1-6,** wherein the biological system comprises living cells.

8. The system or method of any one of claims **1-7,** wherein the one or more analytes comprise small molecules.

9. The system or method of any one of claims **1-8,** wherein the one or more analytes comprise large molecules.

10. The system or method of claim **9,** wherein the one or more analytes comprise polypeptides or proteins.

11. The system or method of claim **9,** wherein the one or more analytes comprise antibodies or functional fragments thereof.

12. The system or method of any one of claims **1-11,** wherein the analytical device is configured for high throughput screening.

13. The system or method of any one of claims **1-12,** wherein the biological assay is RT-CETSA.

14. The system or method of any one of claims **1-13,** wherein the analytical device is capable of simultaneously heating and collecting real time luminescence data for multiple samples, the device comprising:
**(a)** a thermal cycler block adapted to receive a multi-well plate comprising the multiple samples;
**(b)** a detection device capable of detecting luminescence; and
**(c)** a thermal top-heat assembly adapted to maintain even heating across the top of the multi-well plate and to allow a luminescent signal to pass through to the detection device;
wherein the detection device is positioned such that it can detect changing luminescence in the multiple samples in real time over a range of temperature.

15. The system or method of **14,** wherein the detection device is a Charge-Coupled Device (CCD) sensor or a CMOS sensor.

16. The system or method of claim **14** or **15,** wherein the multi-well plate is a 96-well plate, a 384-well plate, or a 1,536-well plate.

## Patentansprüche

1. System zum Analysieren von Daten aus einem biologischen Assay, wobei der biologische Assay ein biologisches System umfasst, und wobei das System zum Analysieren von Daten aus einem oder mehreren Analyten diejenigen Analyten identifiziert, die eine gewünschte Aktivität in dem biologischen System zeigen, wobei Daten aus dem biologischen Assay unter Verwendung einer Analysevorrichtung erhalten werden und wobei die Daten aus dem biologischen Assay Ergebnisse umfassen, die unter einer Vielzahl experimenteller Bedingungen erhalten worden sind, wobei die experimentellen Bedingungen in Bezug auf die getesteten Werte für eine erste unabhängige Variable und die getesteten Werte für eine zweite unabhängige Variable variieren, wobei die getesteten Werte für die erste unabhängige Variable kollektiv einen Temperaturgradienten umfassen und wobei die zweite unabhängige Variable die Konzentration jedes einzelnen der einen oder der mehreren Analyten ist, die in jeder der Vielzahl experimenteller Bedingungen vorhanden ist, und wobei der biologische Assay Real Time Cellular Thermal Shift Assay (RT-CETSA), Differential-Scanning-Fluorimetrie, die Thermal-Shift-Analyse, intrinsische Fluoreszenz-Differential-Scanning-Fluorimetrie oder Nano-Differential-Scanning-Fluorimetrie (nanoDSF) umfasst,
wobei das System umfasst:
einen Speicher, der Anweisungen speichert; und
einen oder mehrere Prozessoren, die als Reaktion auf die Ausführung der Anweisungen konfiguriert sind, um:
**a)** initiale Ergebnisse von der Analysevorrichtung zu empfangen;
**b)** die initialen Ergebnisse zu verarbeiten, um verarbeitete Ergebnisse zu erhalten;
**c)** die verarbeiteten Ergebnisse für jede Konzentration eines einzelnen Analyten an ein erstes Modell und ein zweites Modell bei jedem getesteten Wert der ersten unabhängigen Variablen anzupassen, wobei das erste Modell ein lineares Nullmodell mit einer Steigung von 0 ist und das zweite Modell ein alternatives logarithmisches Modell ist;
**d)** eine Vielzahl von Residualquadratsummen (RSS)-Werten für das erste und zweite Modell bei jedem getesteten Wert der ersten unabhängigen Variablen zu bestimmen;
**e)** die Vielzahl von RSS-Werten unter Verwendung eines nicht-parametrischen Anpassungsgüte-Tests für jeden getesteten Wert der ersten unabhängigen Variablen zu analysieren;
**f)** als Reaktion auf die Feststellung für den einzelnen Analyten, dass das zweite Modell für mindestens einen getesteten Wert der ersten unabhängigen Variable eine bessere Anpassung darstellt als das erste Modell, den einzelnen Analyten als einen, der die gewünschte Aktivität in dem biologischen System aufweist zu identifizieren; und
**g)** die Schritte (c) bis (f) für jeden der einen oder mehreren Analyten zu wiederholen.

2. Verfahren zum Analysieren von Daten aus einem biologischen Assay, wobei der biologische Assay ein biologisches System umfasst und wobei das System zum Analysieren von Daten aus einem oder mehreren Analyten diejenigen Analyten identifiziert, die eine gewünschte Aktivität in dem biologischen System zeigen, wobei Daten aus dem biologischen Assay unter Verwendung einer Analysevorrichtung erhalten werden und wobei die Daten aus dem biologischen Assay Ergebnisse umfassen, die unter einer Vielzahl experimenteller Bedingungen erhalten worden sind, wobei die experimentellen Bedingungen in Bezug auf die getesteten Werte für eine erste unabhängige Variable und die getesteten Werte für eine zweite unabhängige Variable variieren, wobei die getesteten Werte für die erste unabhängige Variable kollektiv einen Temperaturgradienten umfassen und wobei die zweite unabhängige Variable die Konzentration jedes einzelnen der einen oder der mehreren Analyten ist, die in jeder der Vielzahl experimenteller Bedingungen vorhanden ist, und wobei der biologische Assay Real Time Cellular Thermal Shift Assay (RT-CETSA), Differential-Scanning-Fluorimetrie, die Thermal-Shift-Analyse, intrinsische Fluoreszenz-Differential-Scanning-Fluorimetrie oder Nano-Differential-Scanning-Fluorimetrie (nanoDSF) umfasst,
wobei das Verfahren umfasst:
**a)** Empfangen initialer Ergebnisse von der Analysevorrichtung;
**b)** Verarbeiten der initialen Ergebnisse, um verarbeitete Ergebnisse zu erhalten;
**c)** Anpassen der verarbeiteten Ergebnisse für jede Konzentration eines einzelnen Analyten an ein erstes Modell und ein zweites Modell bei jedem getesteten Wert der ersten unabhängigen Variablen, wobei das erste Modell ein lineares Nullmodell mit einer Steigung von 0 ist und das zweite Modell ein alternatives logarithmisches Modell ist;
**d)** Bestimmen einer Vielzahl von RSS-Werten für das erste und zweite Modell bei jedem getesteten Wert der ersten unabhängigen Variablen;
**e)** Analysieren der Vielzahl von RSS-Werten unter Verwendung eines nicht-parametrischen Anpassungsgüte-Tests für jeden getesteten Wert der ersten unabhängigen Variablen;
**f)** als Reaktion auf die Feststellung für den einzelnen Analyten, dass das zweite Modell für mindestens einen getesteten Wert der ersten unabhängigen Variable eine bessere Anpassung darstellt als das erste Modell, den einzelnen Analyten als einen, der die gewünschte Aktivität in dem biologischen System aufweist zu identifizieren; und
**g)** Wiederholen der Schritte (c)-(f) für jeden der einen oder mehreren Analyten.

3. System nach Anspruch **1** oder Verfahren nach Anspruch **2,** wobei der nicht-parametrischen Anpassungsgüte-Test ein Mann-Whitney-U-Test ist.

4. System oder Verfahren nach einem der Ansprüche **1-3,** wobei das alternative logarithmische Modell ein log-logistisches Anpassungsmodell mit 3-5 Parametern ist.

5. System oder Verfahren nach Anspruch **4,** wobei das alternative logarithmische Modell ein log-logistisches Anpassungsmodell mit 4 Parametern ist.

6. System oder Verfahren nach einem der Ansprüche **1- 5,** wobei der eine oder die mehreren Prozessoren ferner so konfiguriert sind, einen EC₅₀-Wert für den einzelnen Analyten zu berechnen, indem die RSS-Werte aus dem ersten und dem zweiten Modell bei jedem Wert der ersten unabhängigen Variablen aufgetragen werden und dann das zweite Modell an die verarbeiteten Ergebnisse bei dem Wert der ersten unabhängigen Variablen anpasst wird, bei dem der Betrag der RSS-Differenz zwischen dem ersten und dem zweiten Modell am größten ist.

7. System oder Verfahren nach einem der Ansprüche **1-6,** wobei das biologische System lebende Zellen umfasst.

8. System oder Verfahren nach einem der Ansprüche **1-7,** wobei der eine oder die mehreren Analyten kleine Moleküle umfassen.

9. System oder Verfahren nach einem der Ansprüche **1-8,** wobei der eine oder die mehreren Analyten große Moleküle umfassen.

10. System oder Verfahren nach Anspruch **9,** wobei der eine oder die mehreren Analyten Polypeptide oder Proteine umfassen.

11. System oder Verfahren nach Anspruch **9,** wobei der eine oder die mehreren Analyten Antikörper oder funktionelle Fragmente davon umfassen.

12. System oder Verfahren nach einem der Ansprüche **1-11,** wobei die Analysevorrichtung für ein Hochdurchsatz-Screening konfiguriert ist.

13. System oder Verfahren nach einem der Ansprüche **1-12,** wobei der biologische Assay RT-CETSA ist.

14. System oder Verfahren nach einem der Ansprüche **1-13,** wobei die Analysevorrichtung in der Lage ist, mehrere Proben gleichzeitig zu erwärmen und Echtzeit-Lumineszenzdaten zu erfassen, wobei die Vorrichtung umfasst:
**(a)** einen Thermocycler-Block, der dafür ausgelegt ist, eine Multi-Well-Platte mit den mehreren Proben aufzunehmen;
**(b)** eine Nachweisvorrichtung, die in der Lage ist, Lumineszenz nachzuweisen; und
**(c)** eine thermische Oberwärme-Baugruppe, die dafür ausgelegt ist, eine gleichmäßige Erwärmung über die Oberseite der Multi-Well-Platte zu gewährleisten und ein Lumineszenzsignal durchzulassen, damit es von der Nachweisvorrichtung erfasst werden kann;
wobei die Nachweisvorrichtung so positioniert ist, dass sie eine sich ändernde Lumineszenz in den mehreren Proben in Echtzeit über einen Temperaturbereich nachweisen kann.

15. System oder Verfahren nach **14,** wobei die Nachweisvorrichtung ein CCD- (Charge-Coupled-Device-) Sensor oder ein CMOS-Sensor ist.

16. System oder Verfahren nach Anspruch **14** oder **15,** wobei die Multi-Well-Platte eine 96-Well-Platte, eine 384-Well-Platte oder eine 1.536-Well-Platte ist.

## Revendications

1. Système d'analyse de données issues d'un essai biologique, sachant que l'essai biologique comprend un système biologique, et sachant que le système d'analyse de données identifie, parmi un ou plusieurs analytes, les analytes qui présentent une activité souhaitée dans le système biologique, sachant que des données issues de l'essai biologique sont obtenues moyennant un appareil analytique, et sachant que les données issues de l'essai biologique comprennent des résultats obtenus dans une pluralité de conditions expérimentales, les conditions expérimentales variant en fonction de valeurs testées pour une première variable indépendante et de valeurs testées pour une deuxième variable indépendante, sachant que les valeurs testées pour la première variable indépendante comprennent collectivement un gradient de température et sachant que la deuxième variable indépendante est la concentration de chacun de l'un ou des plusieurs analytes présents dans chacune de la pluralité de conditions expérimentales, et sachant que l'essai biologique comprend un essai de décalage thermique cellulaire en temps réel (RT-CETSA), une fluorimétrie différentielle à balayage, une analyse de décalage thermique, une fluorimétrie différentielle à balayage par fluorescence intrinsèque, ou une nano fluorimétrie différentielle à balayage (nanoDSF), le système comprenant :
une mémoire stockant des instructions ; et
un ou plusieurs processeurs qui, en réponse à l'exécution des instructions, sont configurés pour :
a) recevoir des résultats initiaux depuis l'appareil analytique ;
b) traiter les résultats initiaux pour obtenir des résultats traités ;
c) ajuster les résultats traités pour chaque concentration d'un analyte unique à un premier et un deuxième modèle à chaque valeur testée de la première variable indépendante, sachant que le premier modèle est un modèle nul linéaire avec une pente de 0, et le deuxième modèle est un modèle logarithmique alternatif ;
d) déterminer une pluralité de valeurs de somme des carrés des résidus (RSS) pour le premier et le deuxième modèle à chaque valeur testée de la première variable indépendante ;
e) analyser la pluralité de valeurs de RSS moyennant un test non paramétrique de qualité d'ajustement à chaque valeur testée de la première variable indépendante ;
f) en réponse à la détermination, pour l'analyte unique, que le deuxième modèle présente un meilleur ajustement que le premier modèle pour au moins une valeur testée de la première variable indépendante, identifier l'analyte unique comme ayant l'activité souhaitée dans le système biologique ; et
g) répéter les étapes (c) à (f) pour chacun de l'un ou des plusieurs analytes.

2. Procédé d'analyse de données issues d'un essai biologique, sachant que l'essai biologique comprend un système biologique, et sachant que le système d'analyse de données identifie, parmi un ou plusieurs analytes, les analytes qui présentent une activité souhaitée dans le système biologique, sachant que des données issues de l'essai biologique sont obtenues moyennant un appareil analytique, et sachant que les données issues de l'essai biologique comprennent des résultats obtenus dans une pluralité de conditions expérimentales, les conditions expérimentales variant en fonction de valeurs testées pour une première variable indépendante et de valeurs testées pour une deuxième variable indépendante, sachant que les valeurs testées pour la première variable indépendante comprennent collectivement un gradient de température et sachant que la deuxième variable indépendante est la concentration de chacun de l'un ou des plusieurs analytes présents dans chacune de la pluralité de conditions expérimentales, et sachant que l'essai biologique comprend une RT-CETSA, une fluorimétrie différentielle à balayage, une analyse de décalage thermique, une fluorimétrie différentielle à balayage par fluorescence intrinsèque, ou une nanoDSF, le procédé comprenant :
a) la réception de résultats initiaux depuis l'appareil analytique ;
b) le traitement des résultats initiaux pour obtenir des résultats traités ;
c) l'ajustement des résultats traités pour chaque concentration d'un analyte unique à un premier modèle et un deuxième modèle à chaque valeur testée de la première variable indépendante, sachant que le premier modèle est un modèle nul linéaire avec une pente de 0, et le deuxième modèle est un modèle logarithmique alternatif ;
d) la détermination d'une pluralité de valeurs de RSS pour le premier et le deuxième modèle à chaque valeur testée de la première variable indépendante ;
e) l'analyse de la pluralité de valeurs de RSS moyennant un test non paramétrique de qualité d'ajustement à chaque valeur testée de la première variable indépendante ;
f) en réponse à la détermination, pour l'analyte unique, que le deuxième modèle présente un meilleur ajustement que le premier modèle pour au moins une valeur testée de la première variable indépendante, l'identification de l'analyte unique comme ayant l'activité souhaitée dans le système biologique ; et
g) la répétition des étapes (c) à (f) pour chacun de l'un ou des plusieurs analytes.

3. Le système de la revendication 1 ou le procédé de la revendication 2, sachant que le test non paramétrique de qualité d'ajustement est un test U de Mann-Whitney.

4. Le système ou procédé de l'une quelconque des revendications 1 à 3, sachant que le modèle logarithmique alternatif est un modèle d'ajustement log-logistique à 3 à 5 paramètres.

5. Le système ou procédé de la revendication 4, sachant que le modèle logarithmique alternatif est un modèle d'ajustement log-logistique à 4 paramètres.

6. Le système ou procédé de l'une quelconque des revendications 1 à 5, sachant que l'un ou les plusieurs processeurs sont en outre configurés pour calculer une valeur EC₅₀ pour l'analyte unique en traçant les valeurs de RSS issues du premier et du deuxième modèle à chaque valeur de la première variable indépendante, puis en ajustant le deuxième modèle aux résultats traités à la valeur de la première variable indépendante pour laquelle l'ampleur de la différence de RSS entre le premier et le deuxième modèle est la plus grande.

7. Le système ou procédé de l'une quelconque des revendications 1 à 6, sachant que le système biologique comprend des cellules vivantes.

8. Le système ou procédé de l'une quelconque des revendications 1 à 7, sachant que l'un ou les plusieurs analytes comprennent des petites molécules.

9. Le système ou procédé de l'une quelconque des revendications 1 à 8, sachant que l'un ou les plusieurs analytes comprennent des grandes molécules.

10. Le système ou procédé de la revendication 9, sachant que l'un ou les plusieurs analytes comprennent des polypeptides ou des protéines.

11. Le système ou procédé de la revendication 9, sachant que l'un ou les plusieurs analytes comprennent des anticorps ou des fragments fonctionnels de ceux-ci.

12. Le système ou procédé de l'une quelconque des revendications 1 à 11, sachant que l'appareil analytique est configuré pour un criblage à haut débit.

13. Le système ou procédé de l'une quelconque des revendications 1 à 12, sachant que l'essai biologique est un RT-CETSA.

14. Le système ou procédé de l'une quelconque des revendications 1 à 13, sachant que l'appareil analytique est apte à simultanément chauffer et collecter des données de luminescence en temps réel pour de multiples échantillons, l'appareil comprenant :
(a) un bloc thermocycleur apte à recevoir une plaque multi-puits comprenant les multiples échantillons ;
(b) un appareil de détection apte à détecter la luminescence ; et
(c) un ensemble thermique supérieur apte à maintenir un chauffage uniforme sur tout le dessus de la plaque multi-puits et à permettre à un signal luminescent de passer à travers l'appareil de détection ;
sachant que l'appareil de détection est positionné de sorte à pouvoir détecter en temps réel un changement de luminescence dans les multiples échantillons sur une plage de température.

15. Le système ou procédé de la revendication 14, sachant que l'appareil de détection est un capteur à dispositif de transfert de charge (CCD) ou un capteur CMOS.

16. Le système ou procédé de la revendication 14 ou 15, sachant que la plaque multi-puits est une plaque à 96 puits, une plaque à 384 puits, ou une plaque à 1 536 puits.
